# EUROPEAN PATENT APPLICATION

(11) **EP 1 798 225 A1**
(43) Date of publication of application: **20.06.2007**
(21) Application number: 05783620.7
(22) Date of filing: 16.09.2005
(51) Int. Cl.: C07D 239/54, A61K 31/513, A61P 43/00, C07D 239/47, C07D 473/18, C07D 473/34, C07H 21/04, C07D 473/40, A61K 31/7125

(54) **NUCLEOSIDE ANALOGUES OR SALTS THEREOF**

(30) Priority: 16.09.2004 JP 2004270103
(71) Applicant: Gifu University, Gifu-shi Gifu 5011193 (JP)
(72) Inventor: YUKIO, Kitade c/o Gifu University, Gifu 501-1193 (JP); UENO, Yoshihito c/o Gifu University, Gifu 501-1193 (JP)
(74) Representative: Vossius & Partner
(86) International application number: PCT/JP2005/017168
(87) International publication number: WO 2006/030906

(57) **Abstract**

It is an object to provide a nucleoside analog that can produce an oligonucleotide analog in which the two properties of chemical and biological stability, and the ability to form double strands, are excellent, and an oligonucleotide analog that includes that nucleoside analog.
This is achieved by a nucleoside analog or salt thereof represented by Formula (1) below, in which, in Formula (1), R¹ is any group selected from the group consisting of the group of Formula (1), the group of Formula (2), the group of Formula (3), the group of Formula (4), the group of Formula (5), the group of Formula (6), the group of Formula (7), the group of Formula (8), and any of these groups whose functional group has been protected by a protecting group, and k, l, m, and n are each independently an integer from 1 to 10.

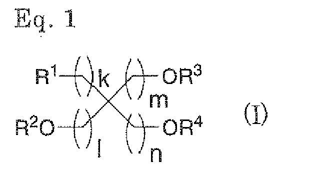

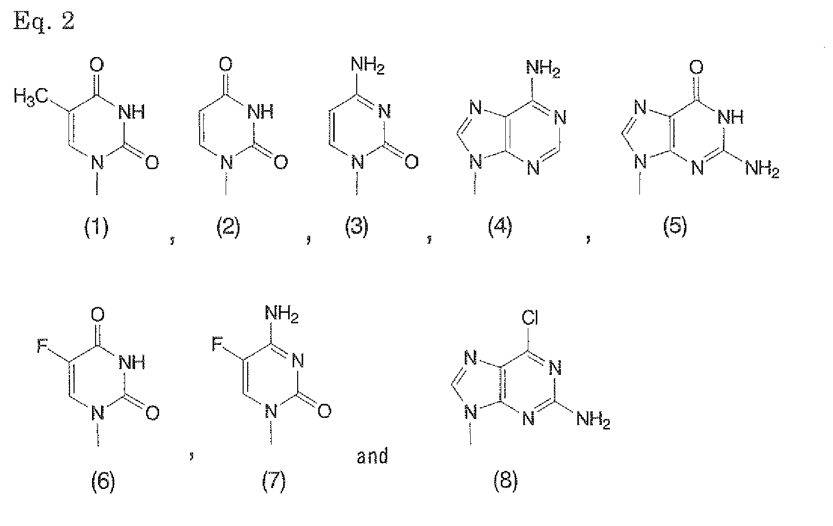

SEQUENCE LISTING

<110> Gifu University

<120> Nucleoside analogues and salt thereof

<130> H2486

<150> JP2004-270103
<151> 2004-09-16

<160> 3

<170> PatentIn version 3.1

<210> 1
<211> 17
<212> DNA
<213> Artificial

<220>
<223> A of 8th base is replaced with the nucleoside analogue.

<400> 1
aaggaaaaga ggaaaga 17

<210> 2
<211> 17
<212> DNA
<213> Artificial

<220>
<223> correspondence of oligonucleotide 1

<400> 2
tctttcctct tttcctt 17

<210> 3
<211> 17
<212> DNA
<213> Artificial

<220>
<223> correspondance of oligonucleotide 1

<400> 3

aaggaaaaga ggaaaga 17

## Description

### Technical Field

The present invention relates to nucleoside analogs or salts thereof.

### Background Art

In recent years, the methodology of targeting genetic information itself for therapy has become increasingly widespread. One such methodology is the antisense method, which employs oligonucleotides.
In the antisense method, a chemically synthesized oligonucleotide analog (such as single-strand DNA composed of 15 to 20 base pairs) is added to a cell to form a DNA etc./mRNA double-stranded nucleic acid with a target messenger RNA (mRNA) in order to effect base sequence-specific inhibition of the expression of a target gene and thereby impede the process of translation from mRNA to protein. With the antisense method, it is possible to logically design and synthesize an antisense molecule if the base sequence of the virus or gene causing the disease is already known, and thus the antisense method holds potential as an effective method of treatment for genetic diseases and diseases with various viral origins, which up to now have been considered difficult to cure.

More recently, methods that employ RNAi (RNA interference) have drawn attention as methods for inhibiting gene expression using oligonucleotides. RNAi refers to the phenomenon of introducing double-stranded RNA into a cell to degrade and cleave RNA originating from a chromosome of the cell that has a homologous base sequence.
The mechanism of RNAi currently is thought to be as follows. First, long-chain double-stranded RNA is hydrolyzed by an enzyme called Dicer into a double-stranded RNA about 21 bases long with a 3' -UU dangling end (this is known as siRNA (short interfering RNA)). The siRNA forms an RNA/mRNA double-stranded nucleic acid with a target mRNA, and a cellular protein that recognizes this double-stranded nucleic acid (RISC (RNA-induced Silencing Complex)) binds the double-stranded nucleic acid, and the target mRNA is cleaved by this conjugate. In most cases, this method of using RNAi produces an effect comparable to that of antisense method, with an RNA concentration of about 1/100 that of the antisense method. Consequently, methods that utilize RNAi also have shown increasing promise as effective methods for treatment of genetic diseases and diseases with varying viral causes, which up to now have been considered difficult to cure.

There has been the problem that oligonucleotide analogs containing a ribose ring, which is present in natural oligonucleotides, are extremely chemically and biologically unstable for use in the antisense method and methods that utilize RNAi, for example (for example, see Non-Patent Document 1). On the other hand, to be used in the antisense method and methods that utilize RNAi, for example, there is a need for oligonucleotide analogs that can form a double strand with natural oligonucleotides, but there is the problem that chemically and biologically stable oligonucleotide analogs normally have a poor ability to form double strands (for example, see Non-Patent Document 2). Thus, there has been the problem that it is difficult to achieve a balance between the property of being chemically and biologically stable and the property of having an excellent ability to form a double strand.
However, if an oligonucleotide analog is to be used in DNA chips and gene diagnostic agents, for example, then in order to obtain stable diagnostic results, there also has been a desire for oligonucleotide analogs in which these properties are excellent.
Non-Patent Document 1: Eugen Uhlmann and Anusch Peyman, "Antisense oligonucleotides: a new therapeutic principle," Chemical Reviews, 90:543, 1990.
Non-Patent Document 2: Jin yan Tang, Jamal Temsamani and Sudhir Agrawal, "Self- stabilized antisense oligonucleotide phosphorothioates: properties and anti-HIV activity," Nucleic Acids Research, 21:2729, 1993.

### Disclosure of Invention

### Problem to be Solved by the Invention

Accordingly, it is an object of the present invention to provide a nucleoside analog that can produce an oligonucleotide analog in which the two properties of chemical and biological stability and the ability to form double strands are excellent, and an oligonucleotide analog that includes this nucleoside analog.

### Means for Solving Problem

The invention is a nucleoside analog or salt thereof represented by Formula (I) below.

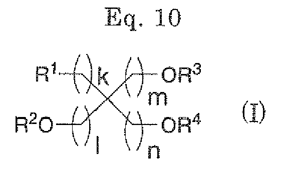

In Formula (I), R¹ is any group selected from the group consisting of the group of Formula (1) below, the group of Formula (1) below in which a functional group of Formula (1) has been protected by a protecting group, the group of Formula (2) below, the group of Formula (2) below in which a functional group of Formula (2) has been protected by a protecting group, the group of Formula (3) below, the group of Formula (3) below in which a functional group of Formula (3) has been protected by a protecting group, the group of Formula (4) below, the group of Formula (4) below in which a functional group of Formula (4) has been protected by a protecting group, the group of Formula (5) below, the group of Formula (5) below in which a functional group of Formula (5) has been protected by a protecting group, the group of Formula (6) below, the group of Formula (6) below in which a functional group of Formula (6) has been protected by a protecting group, the group of Formula (7) below, the group of Formula (7) below in which a functional group of Formula (7) has been protected by a protecting group, the group of Formula (8) below, and the group of Formula (8) below in which a functional group of Formula (8) has been protected by a protecting group;
R² is H or a protecting group;
R³ is H or a protecting group;
R⁴ is H or a solid-phase synthesis activating phosphate group; and
k, 1, m, and n are each independently an integer from 1 to 1.0.

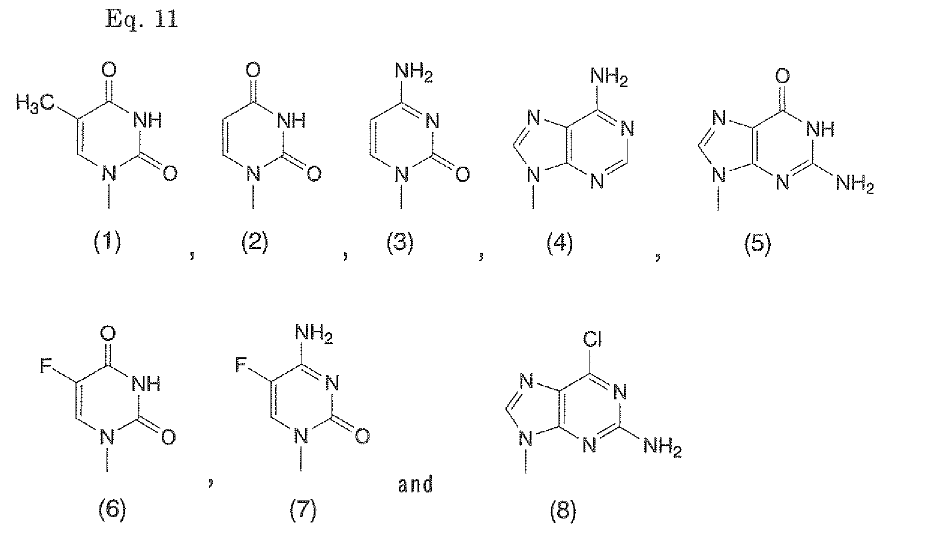

### Effects of the Invention

The present invention designs a novel chemical structure that heretofore has not existed, and was arrived at based on successfully producing a nucleoside analog that has this chemical structure. With this nucleoside analog, the invention can provide an oligonucleotide analog in which the two properties of nuclease resistance and the ability to form a double strand are excellent.

### Brief Description of Drawings

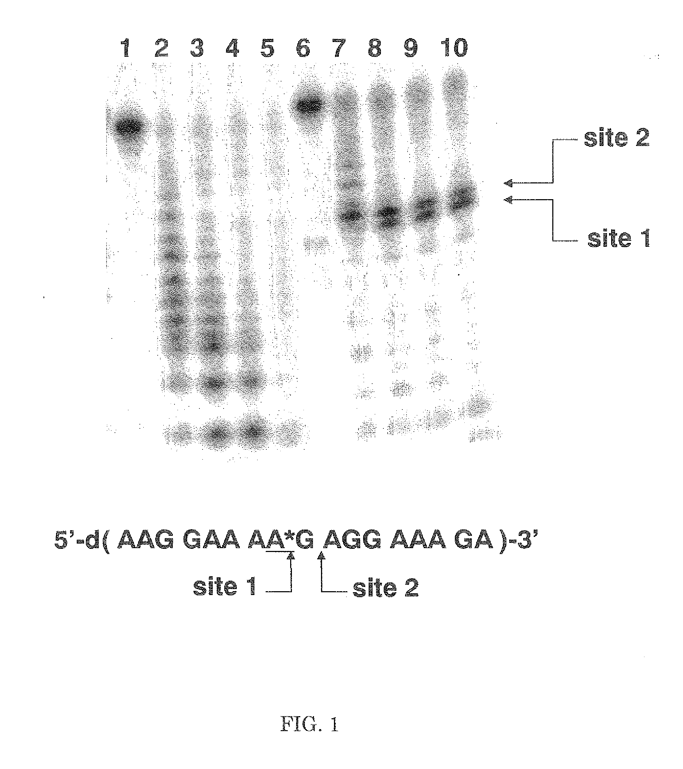

FIG. 1 is a diagram showing the nuclease resistance of an example of the oligonucleotide analog of the invention and an example of the oligonucleotide of the comparative example. Lane 1: natural type 0 min, Lane 2: natural type 5 min, Lane 3: natural type 10 min, Lane 4: natural type 15 min, Lane 5: natural type 30 min, Lane 6: modified type 0 min, Lane 7: modified type 5 min, Lane 8: modified type 10 min, Lane 9. modified type 15 min, Lane 10: modified type 30 min

### Best Mode for Carrying Out the Invention

In the invention, "oligonucleotide" refers to a polymer of nucleoside subunits, for example, and although there is no particular limitation with regard to the number of subunits, it may be 3 to 100 subunits, for example. Here, if the nucleotide analog of the invention is DNA, then the number of subunits is preferably 3 to 100 and more preferably 3 to 30, and if RNA, then the number of subunits is preferably 3 to 50 and more preferably 3 to 30. It should be noted that there are no particular limitations regarding the "oligonucleotide analog" in the invention, so long as nucleoside has been substituted with the nucleoside analog of the invention. For example, nucleosides other than the nucleoside analog of the invention may have a sugar part or a base part that is an analog that is widely known by those skilled in the art.

In the invention, for R¹, a protecting group for protecting the functional group is selected from protecting groups that are widely known within the field of nucleic acid chemistry. For example, it is possible to use benzoyl (Bz), isobutyryl (iBu), phenoxyacetyl (Pac), allyloxycarbonyl (AOC), N,N-dimethylaminomethylene, and acetyl (Ac), for example, as the protecting group.

In the invention, the protecting groups of R² and R³ may be primary alcohol protecting groups that conventionally have been known to the public. Examples of such a protecting group include 4,4'-dimethoxytrityl (DMTr), tert-butyldimethylsilyl (TBDMS), 4-monomethoxytrityl (MMTr), tert-butyldiphenylsilyl (TBDPS), and (9-phenyl)xanthene-9-yl[pixyl].

In the invention, the protecting groups for R¹, R², and R³ can be selected suitably in consideration of the conditions for ultimately producing an oligonucleotide analog that employs the nucleoside analog of the invention. For example, when producing the oligonucleotide analog, it is possible to use a nucleoside analog in which R¹, R², and R³ have been selected suitably in accordance with the conditions under which the protecting groups will ultimately be removed.

In the invention, R⁴ may be a phosphate group conventionally widely known in solid-phase synthesis as a solid-phase synthesis activating phosphate group, and examples thereof include phosphate groups that can form phosphoroamidite, phosphonate, or thiophosphite, for example. An example of a solid-phase synthesis activating phosphate group that forms phosphoroamidite is the group represented by Formula (10) below.

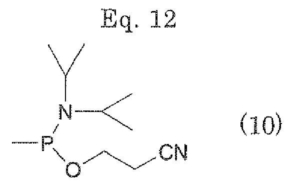

In the invention, salts refer to salts with inorganic bases, salts with organic bases, salts with inorganic acids, and salts with organic acids, for example. Examples of salts with inorganic bases include alkali metal salts such as sodium salts and potassium salts; alkaline earth metal salts such as calcium salts and magnesium salts; and aluminum salts and ammonium salts. Examples of salts with organic bases include salts with trimethylamine, triethylamine, pyridine, picoline, ethanolamine, diethanolamine, triethanolamine, dicyclohexylamine, and N,N'-dibenzylethylenediamine. Examples of salts with inorganic acids include salts with hydrochloric acid, hydrobromic acid, nitric acid, sulfuric acid, and phosphoric acid. Examples of salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid. In this invention, the salts preferably are pharmacologically acceptable salts.

In this invention, k, l, m, and n are each independently an integer from 1 to 10, and preferably an integer from 1 to 6. It is possible for k, l, m, and n to be identical or different.

In the nucleoside analog or the salt thereof represented by Formula (I) of the invention, it is preferable that R² is H, 4,4'-dimethoxytrityl (DMTr), tert-butyldimethylsilyl (TBDMS), 4-monomethoxytrityl (MMTr), tert-butyldiphenylsilyl (TBDPS), or (9-phenyl)xanthene-9-yl, R³ is H, 4,4'-dimethoxytrityl (DMTr), tert-butyldimethylsilyl (TBDMS), 4-monomethoxytrityl (MMTr), tert-butyldiphenylsilyl (TBDPS), or (9-phenyl)xanthene-9-yl, and R⁴ is H or the group represented by Formula (10) below.

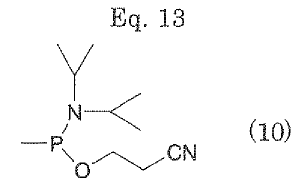

With regard to the nucleoside analog or the salt thereof represented by Formula (I) of the invention, in Formula (I), it is preferable that R¹ is a group selected from the group consisting of the group of Formula (1), the group of Formula (2), the group of Formula (3), the group of Formula (4), the group of Formula (5), the group of Formula (6), the group of Formula (7), and the group of Formula (8), and that k, 1, m, and n are each 1.

It is more preferable that the nucleoside analog or the salt thereof represented by Formula (I) of the invention is
a nucleoside analog represented by Formula (I), in which R¹ is the group of Formula (1) and R², R³, and R⁴ are each H,
a nucleoside analog represented by Formula (I), in which R¹ is the group of Formula (2) and R², R³, and R⁴ are each H,
a nucleoside analog represented by Formula (I), in which R¹ is the group of Formula (3) and R², R³, and R⁴ are each H,
a nucleoside analog represented by Formula (I), in which R¹ is the group of Formula (4) and R², R³, and R⁴ are each H,
a nucleoside analog represented by Formula (I), in which R¹ is the group of Formula (5) and R², R³, and R⁴ are each H,
a nucleoside analog represented by Formula (I), in which R¹ is the group of Formula (6) and R², R³, and R⁴ are each H,
a nucleoside analog represented by Formula (I), in which R¹ is the group of Formula (7) and R², R³, and R⁴ are each H, and
a nucleoside analog represented by Formula (I), in which R¹ is a group in which the functional group of the group of Formula (7) is protected by benzoyl, R² is 4,4'-dimethoxytrityl (DMTr), R³ is tert-butyldiphenylsilyl (TBDPS), and R⁴ is the group represented by Formula (10) below.

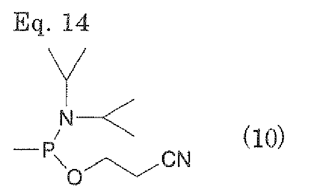

It should be noted that the nucleoside analog or its salt according to the invention is not limited to use for the production of the oligonucleotide analog of the invention, and it can be adopted for other applications as well.

The oligonucleotide analog of the invention is an oligonucleotide analog in which one or more of the nucleosides making up the oligonucleotide have been substituted with a nucleoside analog, wherein the nucleoside analog is the nucleoside analog of the invention in which, in Formula (1), R¹ is any group selected from the group consisting of the group of Formula (1), the group of Formula (2), the group of Formula (3), the group of Formula (4), the group of Formula (5), the group of Formula (6), the group of Formula (7), and the group of Formula (8), and R², R³, and R⁴ are each H. For example, an oligonucleotide analog in which all of the nucleosides making up the oligonucleotide have been substituted with the nucleoside analog is represented by the following formula.

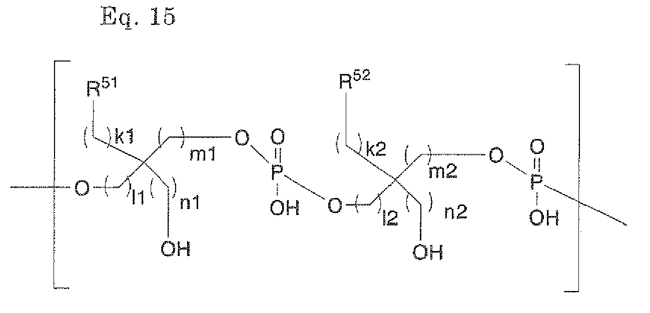

In this formula, R⁵¹ and R⁵² are each independently any group selected from the group consisting of the group of Formula (1), the group of Formula (2), the group of Formula (3), the group of Formula (4), the group of Formula (5), the group of Formula (6), the group of Formula (7), and the group of Formula (8), and k1 k2, l1, l2, m1, m2, n1, and n2 are each independently an integer from 1 to 10.

The oligonucleotide analog of the invention can be a single-strand oligonucleotide or a double-stranded oligonucleotide, for example. If the oligonucleotide analog is double stranded, then one or more of the nucleosides making up one or both single strand oligonucleotide of the double-stranded oligonucleotide is a nucleoside analog in which, in Formula (I), R¹ is any group selected from the group consisting of the group of Formula (1), the group of Formula (2), the group of Formula (3), the group of Formula (4), the group of Formula (5), the group of Formula (6), the group of Formula (7), and the group of Formula (8), and R², R³, and R⁴ are each H.

If the oligonucleotide analog is single stranded, then the oligonucleotide analog of the invention preferably has the ability to form a double strand. This is because the oligonucleotide analog of the invention can be used for antisense and gene detection, for example, if it has the ability to form a double strand with a natural oligonucleotide.

It is preferable that the oligonucleotide analog of the invention is nuclease resistant. This is because digestion by nuclease can be prevented when the oligonucleotide analog of the invention is incorporated into a cell, and thus the activity of the oligonucleotide analog within the cell can be sustained,

The gene expression inhibiting agent of the invention includes the oligonucleotide analog of the invention. With such a gene expression inhibiting agent, the oligonucleotide analog functions as siRNA or antisense, for example, and cleaves the mRNA of a target gene or forms a double strand with the mRNA of a target gene, and as a result, can inhibit gene expression.

The pharmaceutical composition of the invention is for treating diseases that are the result of expression of a gene, and include the gene expression inhibiting agent. In the case of a disease that results from expression of a gene, such as a disease that occurs due to the expression of a certain protein, the pharmaceutical composition inhibits the expression of that gene, and can be used to treat diseases that result from the expression of that gene.

The test kit of the invention includes the oligonucleotide analog of the invention, and tests a gene through the hybridization of the oligonucleotide analog with the gene in the specimen. Examples of such a kit include DNA chips, DNA microarrays, and the like. In addition to the oligonucleotide analog of the invention, this kit also includes a fixing support such as a plate, fiber, or biochip on which a well and the oligonucleotide analog, etc., are fixed. The kit may also include drugs, a coloring reagent that produces color when reacted, and a detection reagent that facilitates detection, for example, in addition to the oligonucleotide analog, etc.

Examples of the DNA chip in general include DNA chips obtained by spotting to fix a solution that includes the oligonucleotide analog of the invention that uses a known gene sequence on a glass substrate, or those obtained by synthesizing and thereby fixing the oligonucleotide analog of the invention on a glass substrate. The DNA chip can detect whether or not there has been expression of a target gene by detecting, through fluorescent pigmentation, for example, hybridization between a gene and the oligonucleotide analog on the substrate after that gene in a specimen is applied to an analysis portion on which the oligonucleotide analog has been fixed. Such a DNA chip for example permits effective analysis even when there is a small amount of reagent, and because many types of DNA probes can be fixed on a single substrate, it is possible to perform multiple analyses based on the same specimen on a single DNA chip.

The method of inhibiting gene expression of the invention uses an oligonucleotide analog to inhibit the expression of a gene. With this method, the oligonucleotide analog functions as siRNA or antisense, for example, and cleaves the mRNA of a target gene or forms a double strand with the mRNA of a target gene, and as a result can inhibit gene expression.

Next, a production method for producing the nucleoside analog of the invention is described using examples. This production method makes it possible to produce the nucleoside analog of the invention, which has a chemical structure that heretofore has not existed.

First is described an example of a method for producing a nucleoside analog or a salt thereof that is represented by the Formula (II) below corresponding to Formula (1) in which R¹ is any group that is selected from the group consisting of the group of Formula (1), the group of Formula (1) in which a functional group of Formula (1) has been protected by a protecting group, the group of Formula (2), the group of Formula (2) in which a functional group of Formula (2) has been protected by a protecting group, the group of Formula (3), the group of Formula (3) in which a functional group of Formula (3) has been protected by a protecting group, the group of Formula (4), the group of Formula (4) in which a functional group of Formula (4) has been protected by a protecting group, the group of Formula (5) in which a functional group of Formula (5) has been protected by a protecting group, the group of Formula (6), the group of Formula (6) in which a functional group of Formula (6) has been protected by a protecting group, the group of Formula (7), the group of Formula (7) in which a functional group of Formula (7) has been protected by a protecting group, the group of Formula (8), and the group of Formula (8) in which a functional group of Formula (8) has been protected by a protecting group, and R², R³, and R⁴ are each H.

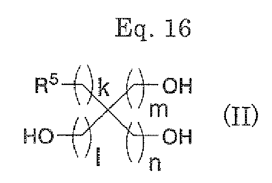

In Formula (II), R⁵ is any group that is selected from the group consisting of the group of Formula (1), the group of Formula (1) in which a functional group of Formula (1) has been protected by a protecting group, the group of Formula (2), the group of Formula (2) in which a functional group of Formula (2) has been protected by a protecting group, the group of Formula (3), the group of Formula (3) in which a functional group of Formula (3) has been protected by a protecting group, the group of Formula (4), the group of Formula (4) in which a functional group of Formula (4) has been protected by a protecting group, the group of Formula (5) in which a functional group of Formula (5) has been protected by a protecting group, the group of Formula (6), the group of Formula (6) in which a functional group of Formula (6) has been protected by a protecting group, the group of Formula (7), the group of Formula (7) in which a functional group of Formula (7) has been protected by a protecting group, the group of Formula (8), and the group of Formula (8) in which a functional group of Formula (8) has been protected by a protecting group, and k, l, m, and n are each independently an integer from 1 to 10.

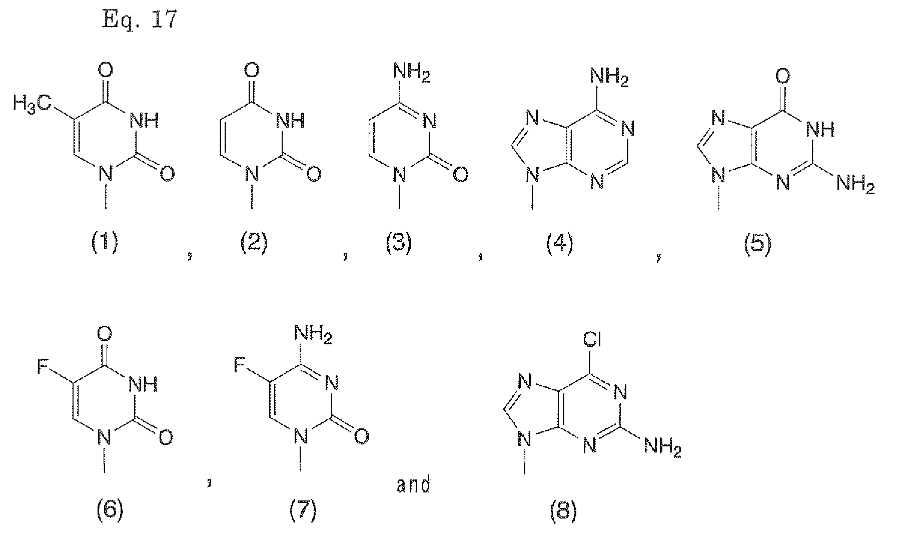

This production method is shown for example in Scheme 1 below. With this production method, the compound represented by Formula (IX) is obtained by reacting the compound represented by Formula (VII) and the compound represented by Formula (VIII), the compound that is represented by Formula (XI) is obtained by condensing the compound represented by Formula (IX) and the compound represented by Formula (X) in the presence of a base, and then R¹¹, R¹², and R¹³ of the compound represented by Formula (XI) are removed to yield the nucleoside analog or the salt thereof that is represented by Formula (II).

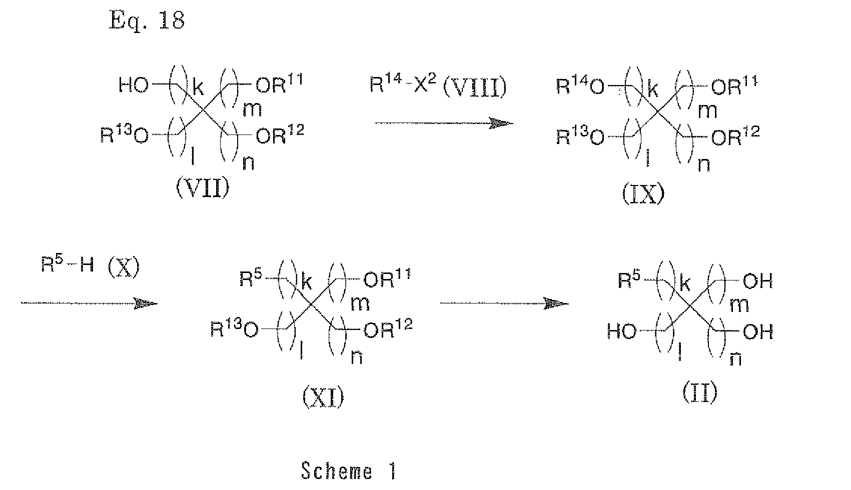

In the formula, R⁵ is any group that is selected from the group consisting of the group of Formula (1), the group of Formula (1) in which a functional group of Formula (1) has been protected by a protecting group, the group of Formula (2), the group of Formula (2) in which a functional group of Formula (2) has been protected by a protecting group, the group of Formula (3), the group of Formula (3) in which a functional group of Formula (3) has been protected by a protecting group, the group of Formula (4), the group of Formula (4) in which a functional group of Formula (4) has been protected by a protecting group, the group of Formula (5) in which a functional group of Formula (5) has been protected by a protecting group, the group of Formula (6), the group of Formula (6) in which a functional group of Formula (6) has been protected by a protecting group, the group of Formula (7), the group of Formula (7), the group of Formula (8), and the group of Formula (8) in which a functional group of Formula (8) has been protected by a protecting group,

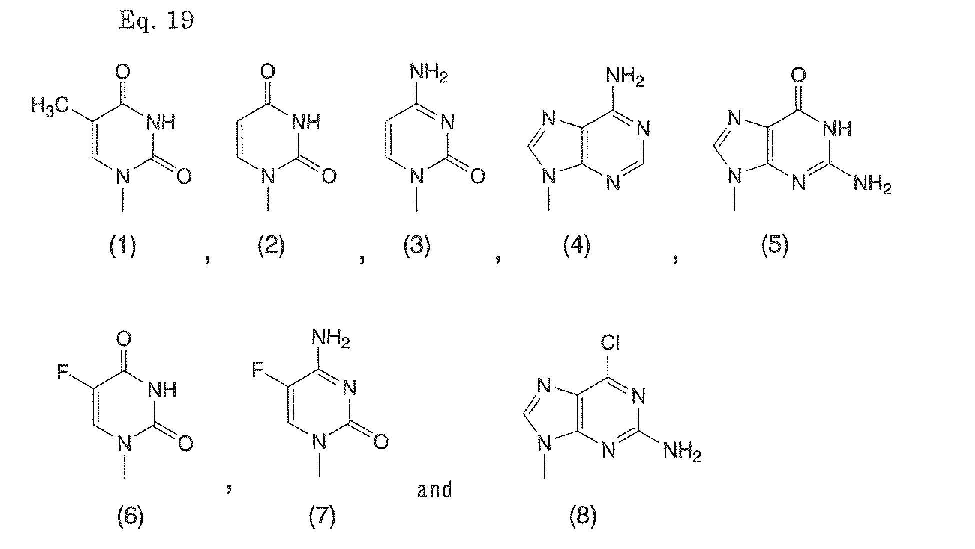

R¹¹ is a protecting group,
R¹² and R¹³ together are a group represented by the formula -CR¹⁵R¹⁶-, R¹⁵ and R¹⁶ are each independently any one selected from the group consisting of a hydrogen atom, a lower alkyl group, and a lower alkoxyl group, R¹⁴ is a group represented by the formula -SO₂-R¹⁷,
R¹⁷ is an aryl group that may be substituted with a lower alkyl,
X² is a halogen atom, and
k, l, m, and n are each independently an integer from 1 to 1.0.

In the invention, as the protecting group for R¹¹ it is possible to use a primary alcohol protecting group conventionally known to the public. Examples of such a protecting group are tert-butyldiphenylsilyl (TBDPS), tert-butyldimethylsilyl (TBDMS), 4,4'-dimethoxytrityl (DMTr), 4-monomethoxytrityl (MMTr), (9-phenyl)xanthene-9-yl[pixyl], acetyl (Ac), and benzoyl (Bz).

In the invention, for R⁵, a protecting group for protecting the functional group is selected from protecting groups that are widely known within the field of nucleic acid chemistry. For example, it is possible to use benzoyl (Bz), isobutyryl (iBu), phenoxyacetyl (Pac), allyloxycarbonyl (AOC), N,N-dimethylaminomethylene, and acetyl (Ac), for example, as that protecting group.

In the invention, the lower alkyl group for R¹⁵ and R¹⁶ is a straight or branched alkyl group, and for example includes 1 to 6 carbon atoms. Examples of the lower alkyl group include methyl, ethyl, n-propyl, isopropyl, n-butyl 2-ethyl butyl, isobutyl, tert-butyl, pentyl, and n-hexyl.

In the invention, the lower alkoxyl group for R¹⁵ and R¹⁶ is a straight or branched alkoxyl group, and for example includes 1 to 6 carbon atoms. Examples of the lower alkoxyl group include methoxy, ethoxy, n-propyloxy, isopropyloxy, n-butyl 2-ethylbutloxy, isobutyloxy, tert-butyloxy, pentyloxy, and n-hexyloxy.

In the invention, examples of the group represented by the formula -CR¹⁵R¹⁶- include -C(CH₃)₂- and -CH(OCH₃)-.

In the invention, the aryl group for R¹⁷ is an aromatic hydrocarbon residue, and for example includes 6 to 30 carbon atoms. Examples of the aryl group include monocyclic aryl groups such as phenyl, and condensed polycyclic aryl groups such as naphthyl, indenyl, and fluorenyl.

In the invention, examples of the aryl group that may be substituted with a lower alkyl for R¹⁷ include aryl groups substituted with 1 to 5 lower alkyls. Specific examples thereof include p-methylphenyl and p-methoxyphenyl.

In the invention, examples of the halogen atom for X² include fluorine atom, chlorine atom, bromine atom, and iodine atom.

In Scheme 1, first, the compound represented by Formula (VII) and the compound represented by Formula (VIII) are reacted, optionally in the presence of a base (such as 4-dimethylamino pyridine (DMAP), DABCO, etc.), yielding the compound represented by Formula (IX). It should be noted that the compound represented by Formula (VIII) can be manufactured with reference to publicly available literature, or can be purchased commercially.

Next, the compound represented by Formula (IX) and the compound represented by Formula (X) are condensed in the presence of a base (such as potassium carbonate, sodium carbonate, rubidium carbonate, lithium carbonate, and cesium carbonate) and optional any crown ether (18-crown-6-ether, 21-crown-7-ether, 15-crown-5-ether, 12-crown-4 -ether, etc.), yielding the compound represented by Formula (XI). It should be noted that the compound represented by Formula (X) can be manufactured with reference to publicly available literature, or can be purchased commercially.

Finally, by removing R¹¹**,** R¹², and R¹³ of the compound represented by Formula (XI), it is possible to obtain the nucleoside analog represented by Formula (II) or its salt. To remove R¹¹, R¹², and R¹³ of the compound represented by Formula (XI), it is possible to select a removal method that is public knowledge in accordance with each of the groups of R¹¹, R¹², and R¹³. For example, if R¹¹ is a silyl group such as tert-butyldiphenylsilyl (TBDPS) or tert-butyldimethylsilyl (TBDMS), then R¹¹ can be removed by processing with tributylammonium fluoride (TBAF) or ammonium chloride. For example, if R¹² and R¹³ are together a group represented by the formula -C(CH₃)₂-, then R¹² and R¹³ can be removed simultaneously by processing with acid (such as trifluoroacetic acid, hydrochloric acid, or acetic acid).

The compound represented by Formula (VII) may also be produced as shown in Scheme 2 below, for example.

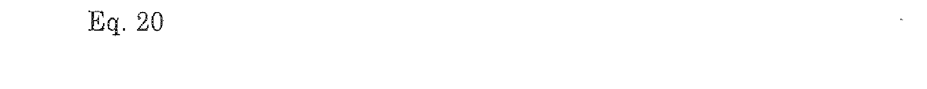

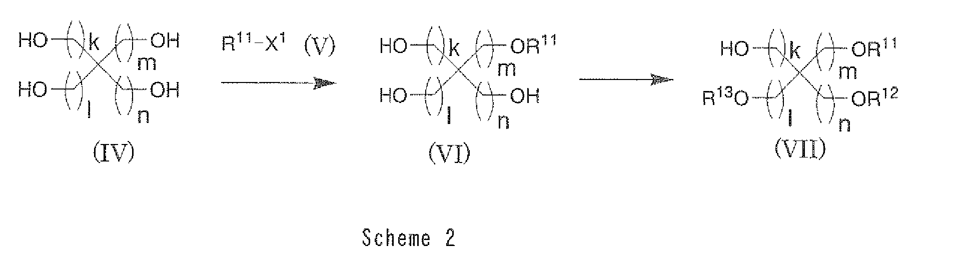

In the formulas, R¹¹ is a protecting group,
R¹² and R¹³ together are a group represented by the formula -CR¹⁵R¹⁶-, R¹⁵ and R¹⁶ are each independently any one selected from the group consisting of a hydrogen atom, a lower alkyl group, and a lower alkoxyl group, X¹ is a halogen atom, and
k, l, m, and n are each independently an integer from 1 to 10.

In the invention, examples of the halogen atom for X¹ include fluorine atom, chlorine atom, bromine atom, and iodine atom.

In Scheme 2, for example, the compound represented by Formula (IV) and the compound represented by Formula (V) can be reacted, optionally in the presence of a base (such as imidazole, DABCO (1,4-Diazabicylo[2.2.2]octane), triethylamine, etc.), to yield the compound represented by Formula (VI). It should be noted that the compound represented by Formula (IV) and the compound represented by Formula (V) can be produced in reference to documents that are available to the public, or can be purchased commercially.

Next, two of the three hydroxyl groups of the compound represented by Formula (VI) can be protected by R¹² and R¹³ to obtain the compound represented by Formula (VII). The method of protecting with R¹² and R¹³ can be selected from protecting methods known to the public, in accordance with the type of the protecting groups R¹² and R¹³. For example, if R¹² and R¹³ together are a group represented by the formula -C(CH₃)₂-, then the compound represented by Formula (VI) can be heated in the presence of acetone and an acid catalyst (such as paratoluene benzoic acid) to obtain the compound of Formula (VII), in which R¹² and R¹³ together are a group represented by the formula -C(CH₃)₂-.

The compound represented by Formula (VII-2) corresponding to Formula (VII) in which k is 2 and l, m, and n are each 1 can be produced as shown in Scheme 3 below, for example.

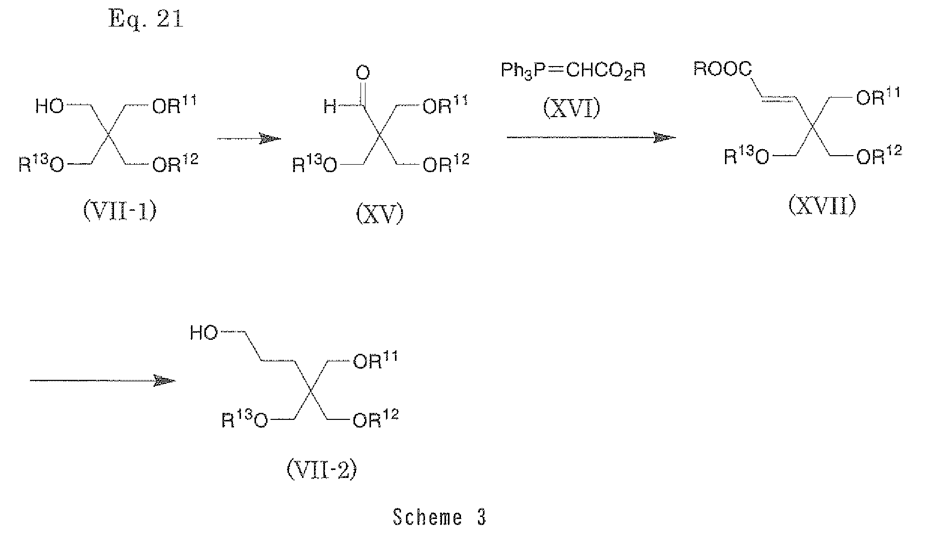

In the formulas,
R¹¹ is a protecting group,
R¹² and R¹³ together are a group represented by the formula -CR¹⁵R¹⁶-, R¹⁵ and R¹⁶ are each independently any one selected from the group consisting of a hydrogen atom, a lower alkyl group, and a lower alkoxyl group, and
R is a lower alkyl group.

In the invention, the lower alkyl group for R is a straight or branched alkyl group, and for example includes 1 to 6 carbon atoms. Examples of the lower alkyl group include methyl, ethyl, n-propyl, isopropyl, n-butyl 2-ethyl butyl, isobutyl, tert-butyl, pentyl, and n-hexyl.

In Scheme 3, first, the compound represented by Formula (VII-1) can be oxidized to obtain the compound represented by Formula (XV). Examples of oxidizing agents include BaMnO₄, Collins reagent [CrO₃(C₅H₅N)₂], PCC (pyridinium chlorochromate) (C₅H₅N⁺HCrO₃Cl⁻), and DCC-DMSO (dicyclohexylcarbodiimide-dimethylsulfoxide). It should be noted that the compound represented by Formula (VII-1), for example, can be the compound of Formula (VII) in Scheme 2, in which k, l, m, and n are each 1. In this case, the compound of Formula (VII), in which k, l, m and n are each 1 can be produced based on the method of production set forth in Scheme 2, using the compound of Formula (IV), in which k, l, m and n are each 1 such as pentaerythritol, as the starting material.

Next, the compound represented by Formula (XV) can be reacted with an ylide compound (XVI) to yield the compound represented by Formula (XVII). The ylide compound (XVI) can be produced through a method of producing an ylide compound in which it is prepared through a method well known to those skilled in the art such as the Wittig reaction or the Horner-Emmons reaction.

Next, the compound represented by Formula (XVII) is reduced catalytically and then, by reducing the ester (-COOR) portion, the compound represented by Formula (VII-2) can be obtained. The catalytic reduction can be carried out by hydrogen gas in the presence of a transition metal catalyst, for example. As the transition metal catalyst, it is possible to use platinum, palladium (such as Pd-C), rhodium (such as Rh₂O₃), ruthenium, or nickel catalysts, for example. Reduction of the ester portion can be carried out using lithium aluminum hydride (LiAlH₄), for example.

It should be noted that it is also possible to produce the compound represented by Formula (VII-2) with reference to documents that are in the public realm, for example. An example of such a document that is available to the public is Satoshi Shuto, Satoshi Niizuma and Akira Matsuda, "One-pot conversion of a, b- unsaturated alcohols into the corresponding carbon-elongated dienes with a stable phosphorus ylide-BaMnO4 system. Synthesis of 6'-methylene derivatives of Neplanocin A as potential antiviral nucleosides. New Neplanocin analogues 11.", The Journal of Organic Chemistry, 63:4489, 1998.

The compound represented by Formula (VII-3) corresponding to Formula (VII) in which m is 2 and k, 1, and n are each 1 can for example be produced as shown in Scheme 4 below.

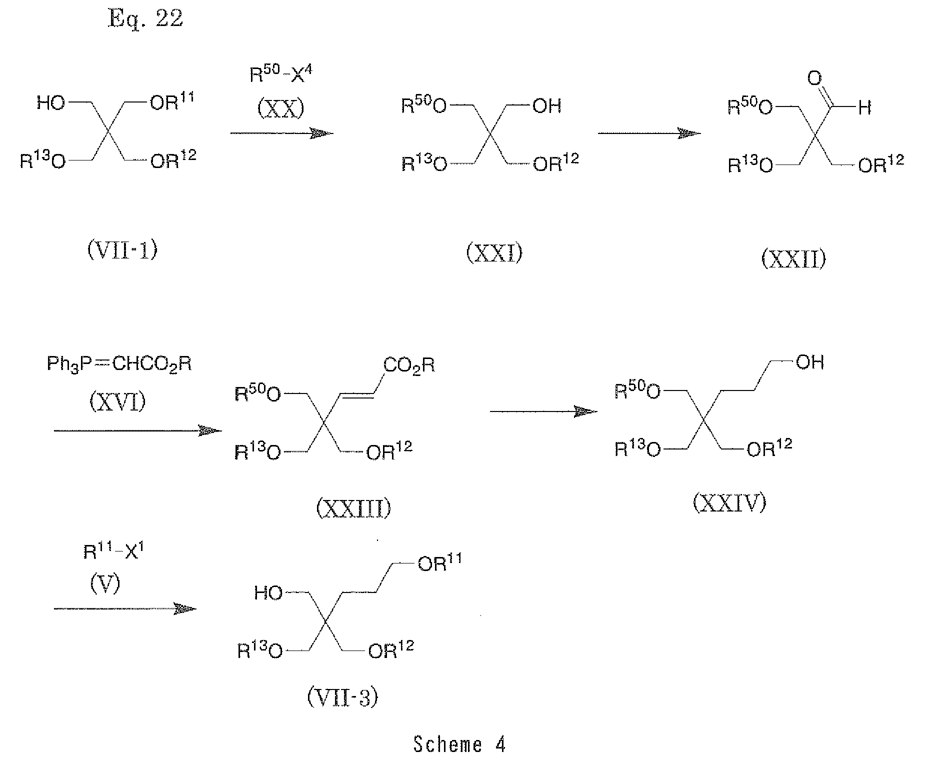

In the formulas, R¹¹ is a protecting group,
R¹² and R¹³ together are a group represented by the formula -CR¹⁵R¹⁶-,
R¹⁵ and R¹⁶ are each independently any one selected from the group consisting of a hydrogen atom, a lower alkyl group, and a lower alkoxyl group,
X¹ and X⁴ are each independently a halogen atom,
R is a lower alkyl group, and
R⁵⁰ is a protecting group.

As the protecting group of R⁵⁰ it is possible to use a primary alcohol protecting group conventionally known to the public. Examples of such a protecting group are tert-butyldiphenylsilyl (TBDPS), tert-butyldimethylsilyl (TBDMS), 4,4'-dimethoxytrityl (DMTr), 4-monomethoxytrityl (MMTr), (9-phenyl)xanthene-9-yl[pixyl], acetyl (Ac), and benzoyl (Bz).

In the invention, examples of the halogen atom for X⁴ include fluorine atom, chlorine atom, bromine atom, and iodine atom.

In Scheme 4, first, the compound represented by Formula (XX) can be reacted with the compound represented by Formula (VII-1), optionally in the presence of a base (for example, imidazole, DABCO, or triethylamine), to yield the compound represented by Formula (XXI). It should be noted that the compound represented by Formula (XX) can be manufactured in reference to documents available to the public, or can be purchased commercially.

Next, the compound represented by Formula (XXI) can be oxidized to obtain the compound represented by Formula (XXII). The oxidation can be performed using the same conditions as when oxidizing the compound represented by Formula (VTT-T) in Scheme 3.

Next, the compound represented by Formula (XXII) can be reacted with a ylide compound (XVI) to obtain the compound represented by Formula (XXTTT). As the ylide compound (XVI) it is possible to use the same compound as the ylide compound (XVI) of Scheme 3.

Next, the compound represented by Formula (XXIII) is reduced catalytically and then, by reducing the ester (-COOR) portion, the compound represented by Formula (XXIV) can be obtained. The catalytic reduction and the reduction of the ester portion can be performed using the same conditions as the catalytic reduction and the ester reduction of the compound represented by Formula (XVII) in Scheme 3.

Next, the compound represented by Formula (XXIV) is reacted with the compound represented by Formula (V) and then the group R⁵⁰ is removed, yielding the compound represented by Formula (VII-3). As for the conditions for the reaction with the compound of Formula (V), it is possible to use the same conditions as the reaction conditions for the compound represented by Formula (IV) and the compound represented by Formula (V) in Scheme 2. The group R⁵⁰ can be removed by selecting a removal method known to the public, in accordance with the group of R⁵⁰.

A compound represented by Formula (VII), in which k, m, 1, and n are integers from 1 to 10, can be produced by techniques that are publicly known, that is, through a combination of protection and deprotection and a homologation reaction after which a number of carbon atoms for the compound increases, the homologation reaction using a ylide compound.

An example of the method for producing the nucleoside analog or its salt represented by Formula (III) below corresponding to Formula (I) in which R¹ is the group of Formula (5) and R², R³, and R⁴ are each H shall be described.

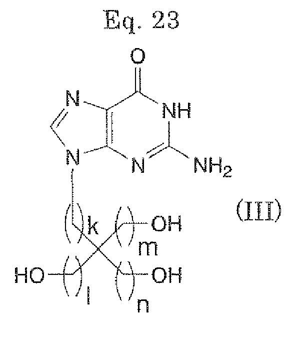

In the formula, k, l, m, and n are each independently an integer from 1 to 10.

This method of production is shown in Scheme 5, for example. With this production method, it is possible to obtain the nucleoside analog, or the salt thereof, represented by Formula (III) by removing R¹¹, R¹², and R¹³ and then hydrolyzing the compound represented by Formula (XIII).

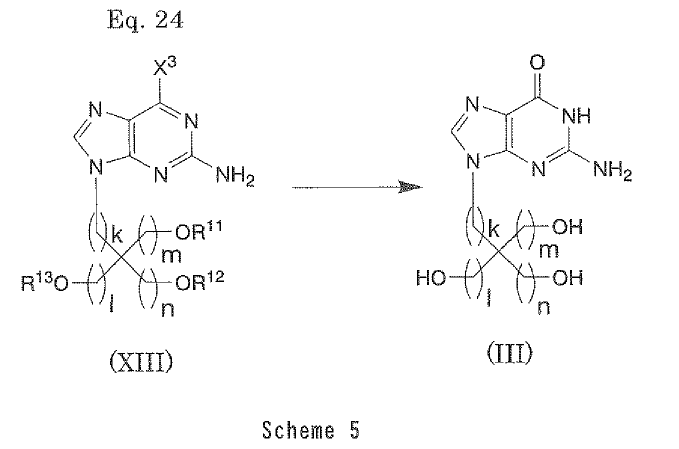

In the formula, R¹¹ is a protecting group,
R¹² and R¹³ together are a group represented by the formula -CR¹⁵R¹⁶-,
R¹⁵ and R¹⁶ are each independently any one selected from the group consisting of a hydrogen atom, a lower alkyl group, and a lower alkoxyl group,
X³ is a halogen atom, and
k, l, m, and n are each independently an integer from 1 to 10.

In the invention, examples of the halogen atom for X³ include fluorine atom, chlorine atom, bromine atom, and iodine atom.
For the removal of R¹¹, R¹², and R¹³ of the compound represented by Formula (XIII), it is possible to select a removal method in the public domain in accordance with each of the groups R¹¹, R¹², and R¹³. For example, if R¹¹ is a silyl group such as tert-butyldiphenylsilyl (TBDPS) or tert-butyldimethylsilyl (TBDMS), then R¹¹ can be removed by processing with tributylammonium fluoride (TBAF) or ammonium chloride. Also, for example, if R¹² and R¹³ are together a group represented by the formula -C(CH₃)₂-, then R¹² and R¹³ can be removed simultaneously by processing with an acid (such as trifluoroacetic acid, hydrochloric acid, or acetic acid).

A removal method that is public knowledge can be selected for the hydrolysis of the compound represented by Formula (XII), For example, it is possible to carry out this hydrolysis by processing with an acid (such as trifluoroacetic acid, hydrochloric acid, or acetic acid). Due to this treatment, for example, if in Formula (XIII), R¹² and R¹³ together are a group represented by the formula -C(CH₃)₂-, then hydrolysis and the removal of R¹² and R¹³ can be carried out simultaneously.

It should be noted that the compound represented by Formula (XIII) explicitly can be manufactured with reference to documents in the public realm, or alternatively, it also can be manufactured explicitly with reference to a method for manufacturing the nucleoside analog of Formula (XI) in Scheme 1, in which R⁵ is the group of Formula (8).

Next is described an example of a method for producing a nucleoside analog, or a salt thereof, that is represented by Formula (XXXIV) below corresponding to Formula (I) in which R¹ is any group selected from the group consisting of the group of Formula (1), the group of Formula (1) in which a functional group of Formula (1) has been protected by a protecting group, the group of Formula (2), the group of Formula (2) in which a functional group of Formula (2) has been protected by a protecting group, the group of Formula (3), the group of Formula (3) in which a functional group of Formula (3) has been protected by a protecting group, the group of Formula (4), the group of Formula (4) in which a functional group of Formula (4) has been protected by a protecting group, the group of Formula (5) in which a functional group of Formula (5) has been protected by a protecting group, the group of Formula (6), the group of Formula (6) in which a functional group of Formula (6) has been protected by a protecting group, the group of Formula (7), the group of Formula (7) in which a functional group of Formula (7) has been protected by a protecting group, the group of Formula (8), and the group of Formula (8) in which a functional group of Formula (8) has been protected by a protecting group, R² is a protecting group, R³ is a protecting group, and R⁴ is a solid-phase synthesis activating phosphate group.

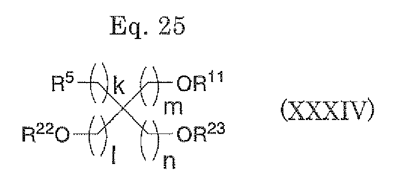

In Formula (XXXIV), R⁵ is any group selected from the group consisting of the group of Formula (1), the group of Formula (1) in which a functional group of Formula (1) has been protected by a protecting group, the group of Formula (2), the group of Formula (2) in which a functional group of Formula (2) has been protected by a protecting group, the group of Formula (3), the group of Formula (3) in which a functional group of Formula (3) has been protected by a protecting group, the group of Formula (4), the group of Formula (4) in which a functional group of Formula (4) has been protected by a protecting group, the group of Formula (5) in which a functional group of Formula (5) has been protected by a protecting group, the group of Formula (6), the group of Formula (6) in which a functional group of Formula (6) has been protected by a protecting group, the group of Formula (7), the group of Formula (7) in which a functional group of Formula (7) has been protected by a protecting group, the group of Formula (8), and the group of Formula (8) in which a functional group of Formula (8) has been protected by a protecting group, R¹¹ is a protecting group, R²² is a protecting group, and R²³ is a solid-phase synthesis activating phosphate group.

In the invention, the protecting group for R²² may be a primary alcohol protecting group conventionally known to the public. Examples of such a protecting group include 4,4'-dimethoxytrityl (DMTr), tert-butyldimethylsilyl (TBDMS), 4-monomethoxytrityl (MMTr), TBDPS and (9-phenyl)xanthene-9-yl[pixyl]

In the invention, for R²³ it is possible to use a phosphate group conventionally known to the public in solid-phase synthesis as the solid-phase synthesis activating phosphate group, and examples thereof include phosphate groups that can form phosphoroamidite, phosphonate, or thiophosphite, for example. An example of a solid-phase synthesis activating phosphate group that forms phosphoroamidite is the group represented by Formula (10) below.

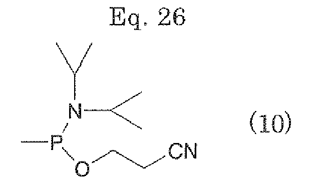

This is produced through the method shown in Scheme 6 below, for example.

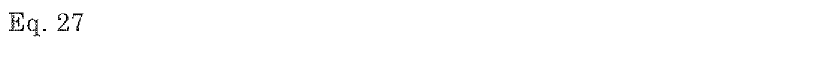

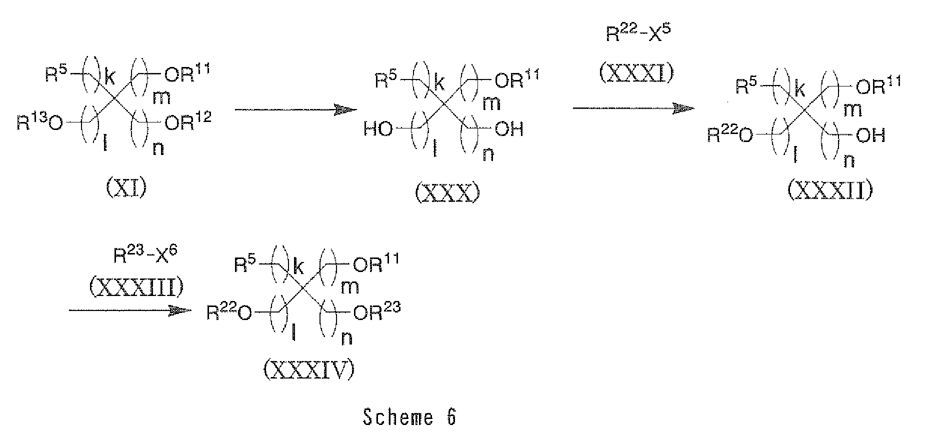

In the formulas, R⁵ is any group selected from the group consisting of the group of Formula (1), the group of Formula (1) in which a functional group of Formula (1) has been protected by a protecting group, the group of Formula (2), the group of Formula (2) in which a functional group of Formula (2) has been protected by a protecting group, the group of Formula (3), the group of Formula (3) in which a functional group of Formula (3) has been protected by a protecting group, the group of Formula (4), the group of Formula (4) in which a functional group of Formula (4) has been protected by a protecting group, the group of Formula (5) in which a functional group of Formula (5) has been protected by a protecting group, the group of Formula (6), the group of Formula (6) in which a functional group of Formula (6) has been protected by a protecting group, the group of Formula (7), the group of Formula (7) in which a functional group of Formula (7) has been protected by a protecting group, the group of Formula (8), and the group of Formula (8) in which a functional group of Formula (8) has been protected by a protecting group, R¹¹ is a protecting group, R²² is a protecting group, R²³ is a solid-phase synthesis activating phosphate group, and X⁵ and X⁶ are halogen atoms.

In the invention, examples of the halogen atom for X⁵ and X⁶ include fluorine atom, chlorine atom, bromine atom, and iodine atom.

With this production method, for example, it is possible to remove R¹² and R¹³ of the compound represented by Formula (XI) to yield the compound that is represented by Formula (XXX). To remove R¹² and R¹³ of the compound represented by Formula (XI), it is possible to select a removal method known to the public, in accordance with each of the groups of R¹² and R¹³. For example, if R¹² and R¹³ together are a group represented by the formula -C(CH₃)₂-, then R¹² and R¹³ can be removed simultaneously by processing with an acid (such as trifluoroacetic acid, hydrochloric acid, or acetic acid).

Next, for example, the compound represented by Formula (XXX) can be reacted with the compound represented by Formula (XXXI), optionally in the presence of a base (such as pyridine) and a catalyst (such as dimethylaminopyridine), to obtain the compound represented by Formula (XXXII). The compound that is represented by Formula (XXXI) can be purchased commercially or explicitly can be manufactured using documents available to the public.

Then, for example, the compound represented by Formula (XXXII) and the compound represented by Formula (XXXIII) can be condensed, optionally in the presence of a base (such as diisopropylethylamine), for example, to obtain the compound represented by Formula (XXXIV).

An example of the method for producing a nucleoside analog, or a salt thereof, represented by Formula (XXXVII) below corresponding to Formula (I) in which R¹ is the group of Formula (5), R² is a protecting group, R³ is a protecting group, and R⁴ is a solid-phase synthesis activating phosphate group is described.

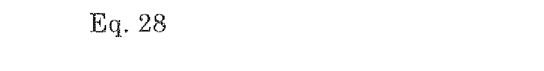

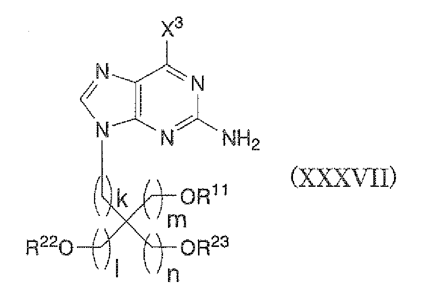

In Formula (XXXVII), R¹¹ is a protecting group, R²² is a protecting group, R²³ is a solid-phase synthesis activating phosphate group, X³ is a halogen atom, and k, 1, m, and n are each independently an integer from 1 to 10.

This is produced through the method shown in Scheme 7 below, for example.

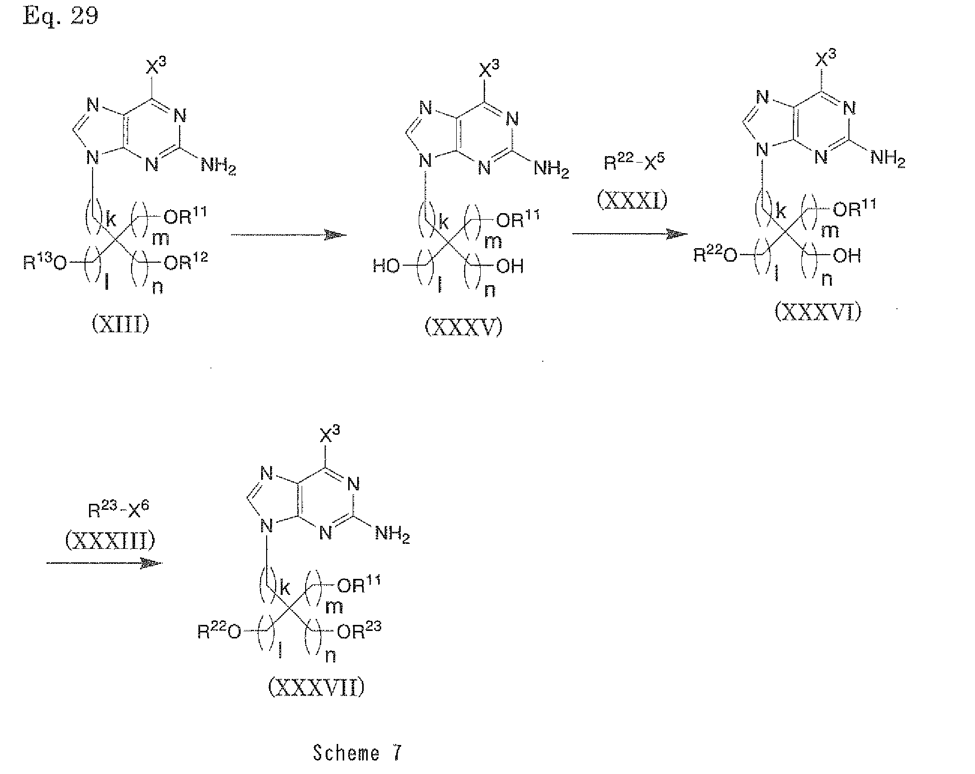

In the formulas, R¹¹ is a protecting group, R²² is a protecting group, R²³ is a solid-phase synthesis activating phosphate group, X³, X⁵, and X⁶ are halogen atoms, and k, 1, m, and n are each independently an integer from 1 to 10.

With this manufacturing method, it is possible, for example, to remove R¹² and R¹³ of the compound represented by Formula (XIII) to yield the compound represented by Formula (XXXV). A removal method known to the public can be selected for the removal of R¹² and R¹³ from the compound represented by Formula (XIII), in accordance with each of the R¹² and R¹³ groups. For example, if R¹² and R¹³ together are a group represented by the formula -C(CH₃)₂-, then R¹² and R¹³ can removed simultaneously by processing with an acid (such as trifluoroacetic acid, hydrochloric acid, or acetic acid).

Next, for example, the compound represented by Formula (XXXV) can be reacted with the compound represented by Formula (XXXI), optionally in the presence of a base (such as pyridine) and a catalyst (such as dimethylaminopyridine), to obtain the compound represented by Formula (XXXVI). The compound that is represented by Formula (XXXI) can be purchased commercially or explicitly can be manufactured using documents known to the public.

Then, for example, the compound represented by Formula (XXXVI) and the compound represented by Formula (XXXIII) can be condensed, optionally in the presence of a base (such as diisopropylethylamine), for example, to obtain the compound represented by Formula (XXXVII).

Next is described an example of a method for producing the oligonucleotide analog of the invention. First, for example, a compound of the nucleoside analog of the invention in which one of three hydroxyl groups is activated with the solid-phase synthesis activating phosphate group, and the remaining two hydroxyl groups are protected is provided. As this compound it is possible to use, for example, the compound represented by Formula (XXXIV) or the compound represented by Formula (XXXVII). As the solid-phase synthesis activating phosphate group it is possible to use a phosphate group conventionally known to the public in solid-phase synthesis, and examples include phosphate groups that form phosphoroamidite, phosphonate, or thiophosphite, for example. The oligonucleotide analog of the invention can be obtained by coupling this activated compound with nucleosides one by one on a solid phase according to the sequence of the oligonucleotide analog using techniques that are conventionally well-known in the field of oligonucleotide synthesis.

It should be noted that it is possible to use nucleosides, coupling reagents, deprotecting reagents, and washing reagents, for example, that are used commonly in nucleic acid solid-phase synthesis. The oligonucleotide analog on the solid-phase carrier that has been obtained is cleaved from the solid-phase carrier, after deprotecting oligonucleotide side chains if necessary, to yield a crude oligonucleotide analog. The reagent that is used for this cleavage can be suitably selected from among reagents that conventionally have been known to the public, according to the solid-phase carrier and the linker (section that joins the solid-phase carrier and the oligonucleotide analog) structure, for example. This crude oligonucleotide analog can also be purified by HPLC, for example, if necessary.

An example of the production in a case where the oligonucleotide analog is double stranded is described next. It is possible, for example, first to produce a single strand oligonucleotide analog through the method discussed above. A single-strand natural oligonucleotide with a sequence complementary to the oligonucleotide analog then is produced separately using a conventional method known to the public. The single-strand oligonucleotide analog that has been obtained is then dissolved in an annealing buffer solution, the single-strand natural oligonucleotide is dissolved in a annealing buffer solution, and these two solutions are, for example, mixed and heated and then gradually cooled to room temperature, yielding a double-stranded oligonucleotide analog. If necessary, the double-stranded oligonucleotide analog can be isolated and purified by further carrying out phenol/chloroform extraction and ethanol precipitation, for example.

It should be noted that in the procedures of Schemes 1 through 7, if necessary it is also possible to introduce protecting groups for the functional groups, deprotect the protecting groups, or change the protecting groups. It should be noted that the selection of protecting groups, the introduction of protecting groups, and the removal of protecting groups, that correspond to the type of the functional group, can be performed in accordance with methods that are public knowledge in this field, and for example, "Protective Groups in Organic Synthesis," T. Greene et al., published by John Wiley & Sons, Inc., for example, can serve as a reference.

### Examples

The following abbreviations are used in the description of this specification.
DMAP: 4'dimethylaminopyridine
TBAF: tributylammonium fluoride
TBDPS-Cl: tert-butyldiphenylsilyl chloride
TFA: Trifluoroacetic acid
Ar: Argon
THF: tetrahydrofuran
TEAA: Triethylammmoium Acetate

### Example 1

Production of 9-(2, 2-dihydroxymethyl-3-hydroxypropyl)adenine

### (1) Production of 1-t-butyl-diphenylsilyloxy-2,2-dihydroxymethyl-3-propanol

Pentaerythritol (3.00 g, 22.02 mmol) and imidazole (3.30 g, 44.04 mmol) were dried and dissolved in DMF (28.5 ml) in an argon atmosphere. TBDPS-Cl (2.22 g, 24.2 mmol) was slowly added dropwise to this solution, and this mixture was agitated for five hours at room temperature. After evaporating the solvent from the mixture, the residue that was obtained was extracted from ethyl acetate and water. The ethyl acetate solution that was extracted was washed with saturated NaCl (aq) and dried with anhydrous sodium sulfate. The solvent was evaporated from that ethyl acetate solution, and the residue that was obtained was purified by silica gel column chromatography
(CHCl₃:MeOH = 1:0 to 20:1), yielding the title compound as a colorless, transparent oil. (yield amount 5.17 g, 13.82 mmol, 62% yield)

¹HNMR (400 MHz, CDCl₃) δ; 7.56 (4H, s, phenyl), 7.32 (6H, s, phenyl), 3.57 (8H, d, J=30.8, CH₂-1,2,2,3), 2.90 (3H, m, OH), 0.98 (9H, s, ter-butyl).

### (2) Production of 2,2-dimethyl-5-t-butyl"diphenylsilyloxylmethyl-5-hydroxymethyl-1,3-dioxane

The 1-t-butyl-diphenylsilyloxy-2,2-dihydroxymethyl-3-propanol (3.50 g, 9.35 mmol) and p-toluene benzoic acid·monohydrate (2.18 g, 11.22 mmol) were dissolved with acetone (100 ml). To this solution was added o-ethyl formate (20 ml), and the mixture was agitated at room temperature for two hours. The mixture was neutralized with dilute ammonia water to stop the reaction, and then the solvent was removed by evaporation. The residue that was obtained was purified by silica gel column chromatography (n-Hex:EtoAc = 20:1 to 5:1), yielding the title compound as a yellow oil. (yield amount 3.76 g, 8.98 mmol, 96% yield)

¹HNMR (400 MHz, CDCl₃) δ; 7.67 (5H, d, J=6.4 Hz, phenyl), 7.40 (5H, q, J=8.0 Hz, phenyl), 3.74 (8H, m, CH2-4,5,5,6), 1.40 (6H, d, J=9.8 Hz, CH₃-2,2), 1.062 (9H, s, ter-butyl).

(3) Production of 2,2-dimethyl-5-t-butyl-diphenylsilyloxylmethyl-5-tolueneslufonylmethyl-1,3-dioxane
DMAP (1.47 g, 12.03 mmol) was added to a CH₂Cl₂ solution (50 ml) of 2,2-dimethyl-5-t-butyl-diphenylsilyloxylmethyl-5-hydroxymethyl-1,3-dioxane (1.66 g, 4.01 mmol), then 5-toluenesulfonylmethyl chloride (2.29 g, 12.03 mmol) was added while chilling with ice and this mixture was agitated for approximately five hours. This mixture was extracted and washed with CH₃Cl and saturated NaHCO₃ (aq). The organic layer that was obtained was dried with sodium sulfate, and the organic solvent was evaporated under reduced pressure. The residue that was obtained was purified by silica gel column chromatography (CHCl₃:MeOH = 100:1 to 20:1), yielding the title compound as a white crystal. (yield amount 1.7787 g, 3.12 mmol, 78% yield)

¹HNMR (400 MHz, CDCl₃) δ: 7.66 (5H, m, phenyl), 7.35 (9H, m, phenyl), 3.82 (4H, s, CH₂-4,6), 3.74 (4H, s, CH₂-5,5), 1.38 (6H, s, CH₃-2,2), 1.05 (9H, s, ter-butyl), 1.05 (3H, s, Ts-Me).

### (4) Production of 2,2-dimethyl-5-t-butyl-diphenylsilyloxylmethyl-5-(adenine-9-yl-methyl)-1,3-dioxane

Adenine (1.65g, 12.2 mmol), potassium carbonate (1.27 g, 9.17 mmol), and 18-crown-6-ether (1.94 g, 7.34 mmol) were added to the 2,2-dimethyl-5-t-butyl-diphenylsilyloxylmethyl-5-toluenesulfonylmethyl-1,3-dioxane (3.48 g, 6.12 mmol), and dried overnight. DMF (150 ml) was added to this mixture, and the mixture that was obtained was heated for 48 hours in a 55°C oil bath. Then, a mixture of n-hexane and ethyl acetate (n-Hex:EtoAc = 1:1) and water were added to that mixture, and extraction was performed. The organic layer that was obtained was washed with saturated saline solution, dried with sodium sulfate, and the solvent was removed by evaporation. The residue that was obtained was purified by silica gel column chromatography (CHCl₃:MeOH = 100:1 to 30:1), yielding the title compound as a white crystal. (yield amount 2.49 g, 4.69 mmol, 76.6% yield)

¹HNMR (400 MHz, CDCl₃) δ; 8.26 (1H, d, J=2.0 Hz, CH-adenine-2), 7.76 (1H, d, J=2.4 Hz, CH-adenine-8), 7.61 (4H, m, phenyl), 7.40 (6H, m, phenyl), 5.85 (2H, s, NH₂-adenine-6), 2.58 (8H, s, CH₂-4,5,5,6), 1.44 (3H, s, CH₃-2), 1.35 (3H, s, CH₃-2), 1.10 (9H, s, ter-butyl)
Mass (EI) m/z: 532.2666 (M⁺) 516, 474, 416, 386, 366.

### (5) Production of 9-(2,2-dihydroxymethyl-3-t-butyl-diphenylsilyloxylmethylpropyl) adenine

TBAF (5 ml) was added to a THF solution (20 ml) of the 2,2- dimethyl- 5-t-butyl-diphenylsilyloxylmethyl-5-(adenine-9-yl-methyl)-1,3-dioxane (1.23 g, 2.32 mmol), and this mixture was agitated for three hours at room temperature. The solvent was evaporated from this mixture under reduced pressure, and the residue that was obtained was purified by silica gel column chromatography (CHCl₃:MeOH = 100: to 10:1), yielding the title compound as a white crystal. (yield amount 0.78 g, 1.59 mmol, 95% yield)

¹HNMR (400 MHz, CDCl₃) δ; 8.04 (1H, s, CH-adenine-2), 7.87 (1H, s, CH-adenine-8), 7.57 (4H, m, phenyl), 7.57 (6H, m, phenyl), 7.18 (2H, s, NH₂-adenine-6), 5.07 (1H, t, J=5.6 Hz, 5-OH), 3.20 (8H, s, CH₂-4,5,5,6), 1.21 (3H, s, CH₃-2), 1.17 (3H, s, CH₃-2), 0.84 (9H, s, ter-butyl);
Mass (EI) m/z: 293.1488 (M⁺) 278, 235, 206, 188, 148;
Anal. calcd for C₁₃H₁₉N₅O₃·1/4 EtOH: C, 53.19; H, 6.78; N, 22.97. found: C, 53.03; H, 6.59; N, 22.94.

### (6) Production of 9-(2,2-dihydroxymethyl-3-hydroxypropyl)-adenine

TFA (1 ml) was added to a THF solution of the 9-(2,2-dihydroxymethyl-3-t-butyl-diphenylsilyloxylmethylpropyl)adenine (30.2 mg, 0.103 mmol), and this mixture was agitated for two hours. Water and methanol were added to this mixture, and then the solvent was removed from the mixture and hardened by drying under reduced pressure. The residue that was obtained was purified by silica gel column chromatography (CHCl₃:MeOH = 30:1 to 10:1), yielding the title compound. (yield amount 18 mg, 0.071 mmol, 69% yield)

¹HNMR (400 MHz, CDCl₃) δ; 8.30 (1H, s, CH-adenine-2), 8.20 (1H, s, CH-adenine-8), 7.56 (2H, s, NH₂-adenine-6), 4.08 (3H, s, OH-2,2,3), 3.24 (8H, m, , CH₂-1,2,2,3);
Mass (EI) m/z: 253.1175 (M⁺) 253, 242, 222, 204, 188;
HRMS (EI) Calcd for C₁₀H₁₅O₃N₅ 253.1175 Found 253.1172.

### Example 2

Production of 9-(2,2-dihydroxymethyl-8-hydroxypropyl)guanine

### (1) Production of 2,2-dimethyl-5-t-Buthyl-diphenyloxylmethyl-5-(2-amino-6-chrolopurine-9-yl-methyl)-1,3-dioxane

Each one of 2,2-dimethyl-5-t-butyl-diphenylsilyloxylmethyl-5-toluenesulfonylmethyl-1,3-dioxane (5.88 g, 10.34 mmol), 2-amino-6-chloropurine (3.51 g, 12.41 mmol), potassium carbonate (2.14 g, 15.51 mmol), and 18-crown-6-ether (3.28 g, 12.41 mmol) were vacuum dried overnight. These were dissolved in DMF (170 ml), and that solution was heated at 55°C for 36 hours. The solvent was evaporated from the reaction mixture, and a mixture of n-hexane and ethyl acetate (n-Hex:EtoAc =1:1) and water were added to the residue that was obtained, and extraction was performed. The organic layer that was obtained was washed with saturated saline solution and dried with sodium sulfate. The solvent was removed from the organic layer by evaporation, and the residue that was obtained was purified by silica gel column chromatography (CHCl₃:MeOH = 100:1 to 20:1), yielding the title compound as a white crystal. (yield amount 2.24 g, 5.27 mmol, 51% yield)

¹HNMR (400 MHz, CDCl₃) δ; 8.02 (1H, s, CH-purine-2), 7.78 (1H, s, CH-purine-8), 7.61 (4H, m, phenyl), 7.40 (6H, m, phenyl), 6.61 (2H, s, NH₂-purine-2), 2.12 (3H, s, OH-3',4',5'), 3.87 (2H, s, CH₂), 3.22 (6H, s, CH₂);
Mass (EI) m/z: 565.2276 (M⁺) 550, 508, 450, 430, 420;
Anal. calcd for C₂₉H₃₆ClN₅O₃Si: C, 61.52; H, 6.41; N, 12.37. found: C, 61.58; H, 6.44; N, 12.24.

### (2) Production of 2,2-dimethyl-5-(2-amino-6-chrolopurine-9-yl-methyl)5-hydroxymethyl-1,3-dioxane

THF (approximately 1 ml) was added to and dissolved the 2,2-dimethyl-5-t-butyl-diphenyloxylmethyl-5-(2-amino-6-chloropurine-9-yl-methyl)-1,3-dioxane (426 mg, 0.783 mmol) under Ar substitution. TBAF (1 ml) was added to the solution, and that mixture was agitated for approximately two hours. The solvent was removed from that mixture by evaporation, and the residue that was obtained was purified by silica gel column chromatography (CHCl₃:MeOH = 100:1 to 20:1), yielding the title compound as a white crystal. (yield amount 216 mg, 0.704 mmol, 90% yield)

¹HNMR (400 MHz, CDCl₃) δ:7.80 (1H, s,
CH-2-amino-6-chloropurine-8), 5.16 (2H, s,
NH₂-2- amino -6-chloropurine -2), 4.43 (2H, s, CH₂), 3.77 (2H, d, J=12.0 Hz, CH₂), 3.58 (2H, d, J=13.0 Hz, CH₂), 1.591 (1H, s, OH), 1.59 (3H, s, CH₃), 1.51 (3H, s, CH₃).

### (3) Production of 9-(2,2-dihydroxymethyl-3-hydroxypropyl)guanine

50% TFA (7.84 ml) was added to the 2, 2-dimethyl- 5-(2-amino-6-chloropurine-9-yl-methyl) 5-hydroxymethyl. 1,3-dioxane (58 mg, 0.187 mmol), and this mixture was agitated for approximately two hours. Water and methanol were added to the mixture, and then the solvent was removed from the mixture and hardened by drying under reduced pressure. The residue that was obtained was purified by silica gel column chromatography (CHCl₃:MeOH = 30:1 to 10:1), yielding the title compound. (yield amount 31 mg, 0.118 mmol, 63% yield)

¹HNMR (400 MHz, CDCl₃) δ; 10.66 (1H, s, NH-1), 7.62 (1H, s, CH-8), 6.61 (2H, s, NH₂-2), 2.12 (3H, s, OH-3',4',5'), 3.87 (2H, s, CH₂), 3.22 (6H, s, CH₂);
Mass (EI) m/z: 269.1124 (M⁺) 269, 238, 220, 191, 165; HRMS (EI) Calcd for 269.1124 Found 269.1118.

### Example 3

Production of 1-(2,2-dihydroxymethyl-3-hydroxypropyl)uracil

### (1) Production of 2,2-dimethyl-5-t-Buthyl-diphenyloxylmethyl-5-(uracil-9-yl-methyl)-1,3-dioxane

Each of 2,2-dimethyl-5-t-butyl-diphenylsilyloxylmethyl-5-toluenesulfonylmethyl-1,3-dioxane (3.65 g, 6.41 mmol), uracil (1.43 g, 12.83 mmol), potassium carbonate (1.06 g, 7.70 mmol), and 18-crown-6-ether (2.04 g, 7.70 mmol) were vacuum dried overnight. These were dissolved in DMF (70 ml) and DMSO (30 ml), and that solution was heated at 55°C for 36 hours. The solvent was evaporated from the reaction mixture, and a mixture of n-hexane and ethyl acetate (n-Hex:EtoAc = 1:1) and water were added to the residue that was obtained, and extraction was performed. The organic layer that was obtained was washed with saturated saline solution and dried with sodium sulfate. The solvent was removed from the organic layer by evaporation, and the residue that was obtained was purified by silica gel column chromatography (CHCl₃:MeOH = 100:1 to 50:1). The colorless, transparent oil compound that was obtained was crystallized with diethyl ether, yielding the title compound as a white crystal. (yield amount 1.62 g, 3.19 mmol, 50% yield)

¹HNMR (400 MHz, CDCl₃) δ; 11.2 (1H, s, NH), 7.63 (4H, m, Phe), 7.44 (7H, m, Phe, CH-uracil-6), 5.45 (1H, d, J=10.4 Hz, CH-uracil-5), 3.09 (8H, m, CH₂-4,5,5,6), 1.31 (3H, s, CH₃-2), 1.20 (3H, s, CH₃-2), 1.00 (9H, s, t-butyl);
Mass (FAB⁺) m/z: 508.2394 (M⁺+H) 451, 307, 289, 154, 107;
Anal. calcd for C₂₈H₃₆N₃O₅Si: C, 66.11; H, 7.13; N, 5.51.
found: C, 66.08; H, 7.03; N, 5.37.

### (2) Production of 2,2-dimethyl-5-(uracil-9-yl-methyl)5-hydroxymethyl-1,3-dioxane

THF (10 ml) was added to and dissolved the 2,2-dimethyl-5-t-butyl-diphenyloxylmethyl-5-(uracil-9-yl-methyl)-1,3-dioxane (200 mg, 0.39 mmol) under Ar substitution. TBAF (3 ml) was added to that solution, and that mixture was agitated overnight. The solvent was removed from that mixture by evaporation, and the residue that was obtained was purified by silica gel column chromatography (CHCl₃:MeOH) = 100:1 to 20:1), yielding the title compound as a white crystal. (yield amount 94 mg, 0.35 mmol, 89% yield)

Mass (EI) m/z: 270.1216 (M⁺) 255, 213, 194, 181, 166;
Anal. calcd for C₁₂H₁₈N₂O₅· 1/6 H₂O: C, 52.74; H, 6.76; N, 10.25. found: C, 52.55; H, 6.43; N, 10.18.

### (3) Production of 1-(2,2-dihydroxymethyl-3-hydroxypropyl)uracil

30% TFA (10 ml) was added to the 2,2-dimethyl-5-(uracil-9-yl-methyl)-5-hydromethyl-1,3-dioxane (34 mg, 0.124 mmol), and this mixture was agitated for approximately two hours. Water and methanol were added to the mixture, and then the solvent was removed from the mixture and hardened by drying under reduced pressure. The residue that was obtained was crystallized from a mixture of n-hexane and ether (n-hexane:Et₂O = 1:1), yielding the title compound as a white crystal. (yield amount 20 mg, 0.087 mmol, 70% yield)

Mass (EI) m/z: 230.0903 (M⁺) 212, 200, 182, 166, 152;
Anal. calcd for C₁₃H₂₀N₂O₅ C, 46.95; H, 6.13; N, 12.17. found: C, 46.82; H, 5.97; N, 11.97.

### Example 4

Production of 1 -(2, 2- dihydroxymethyl- 3 -hydroxypropyl)cytosine

### (1) Production of 2,2-dimethyl-5-t-Buthyl-diphenyloxylmethyl-5-(cytosine-9-yl-methyl)-1,3-dioxane

Each one of 2,2-dimethyl-5-t-butyl-diphenylsilyloxylmethyl 5-toluenesulfonylmethyl-1,3-dioxane (2.80 g, 4.92 mmol), cytosine (1.09 g, 9.84 mmol), potassium carbonate (0.82 g, 5.91 mmol), and 18-crown- 6 -ether (1.56 g, 5.91 mmol) were vacuum dried overnight. These were dissolved in DMF (30 ml) and DMSO (10 ml), and that solution was heated at 60°C for four days. The solvent was evaporated from the reaction mixture, and to the residue that was obtained was added a mixture of n-hexane and ethyl acetate (n-Hex:EtoAc = 1:1) and water, and extraction was performed. The organic layer that was obtained was washed with saturated saline solution and dried with sodium sulfate. The solvent was removed from the organic layer by evaporation, and the residue that was obtained was purified by silica gel column chromatography (CHCl₃:MeOH = 100:1 to 20:1), yielding the title compound as a white crystal. (yield amount 1.20 g, 2.37 mmol, 48% yield)

¹HNMR (400 MHz, CDCl₃) δ: 7.65 (4H, m, phenyl), 7.40 (6H, m, phenyl), 7.26 (1H, s, 2-CH), 5.32 (1H, d, J=6.8 Hz 3-CH), 3.78 (8H, m, CH₂), 1.65 (2H, s, 4-NH₂), 1.43 (3H, s, CH₃-2), 1.34 (3H, s, CH₃-2), 1.11 (9H, s, ter-butyl);
Mass (EI) m/z: 507.2553 (M⁺) 492, 450, 392, 362, 292;
Anal. calcd for C₂₈H₃₇N₃O₄Si· 1/6 Hex: C, 66.72; H, 7.59; N, 8.05. found: C, 66.68; H, 7.53; N, 7.85.

### (2) Production of 2,2-dimethyl-5-(cytosine-9-yl-methyl)5-hydroxymethyl-1,3-dioxane

THF (10 ml) was added to and dissolved the 2,2-dimethyl-5-t-butyl-diphenyloxylmethyl-5-(cytosine-9-yl-methyl)-1,3-dioxane (200 mg, 0.39 mmol) under Ar substitution. TBAF (1 ml) was added to that solution, and the mixture was agitated for two hours. The solvent was removed from the mixture, and the residue that was obtained was purified by silica gel column chromatography (CHCl₃:MeOH = 100:1 to 20:1), yielding the title compound as a white crystal. (yield amount 95 mg, 0.35 mmol, 91% yield)
Mass (EI) m/z: 269.1376 (M⁺) 254, 211, 201, 182, 164.

### (3) Production of 1-(2,2-dihydroxymethyl-3-hydroxypropyl)-cytosine

30% TFA (10 ml) was added to the 2,2-dimethyl-5-(cytosine-9-yl-methyl)5-hydroxymethyl-1,3-dioxane (34 mg, 0.122 mmol), and this mixture was agitated for approximately two hours. Water and methanol were added to the mixture, and then the solvent was removed from the mixture and hardened by drying under reduced pressure. The residue that was obtained was crystallized from a mixture of n-hexane and ether (n-hexane:Et₂O = 1:1), yielding the title compound as a white crystal. (yield amount 20 mg, 0.087 mmol, 69% yield)

¹HNMR (400 MHz, DMSO-d₆) δ: 7.66 (1H, s, 6-CH), 5.85 (1H, s, CH-5), 4.63 (3H, s, OH-2,2,3), 3.69 (2H, s, NH₂-2), 3.24 (8H, s, CH₂-1,2,2,3).

### Example 5

Production of 1-(2,2-dihydroxymethyl-3-hydroxypropyl)thymine

### (1) Production of 2,2-dimethyl-5-t-Buthyl-diphenyloxylmethyl-5-(thymine-9-yl-methyl)-1,3-dioxane

Each one of 2,2-dimethyl-5-t-butyl-diphenylsilyloxylmethyl-5-toluenesulfonylmethyl-1,3-dioxane (2.80 g, 4.92 mmol), thymine (1.24 g, 9.84 mmol), potassium carbonate (0.82 g, 5.91 mmol), and 18-crown-6-ether (1.56 g, 5.91 mmol) were vacuum dried overnight. These were dissolved in DMF (30 ml) and DMSO (10 ml), and that solution was heated at 60°C for four days. The solvent was evaporated from the reaction mixture, and to the residue that was obtained were added a mixture of n-hexane and ethyl acetate (n-Hex:EtoAc= 1:1) and water, and extraction was performed. The organic layer that was obtained was washed with saturated saline solution and dried with sodium sulfate. The solvent was removed from the organic layer by evaporation, and the residue that was obtained was purified by silica gel column chromatography (CHCl₃:MeOH = 100:1 to 20:1), yielding the title compound as a white crystal. (yield amount 1.25 g, 2.41 mmol, 49% yield)

¹HNMR (400 MHz, CDCl₃) δ; 8.16 (1H, s, NH-3), 7.62 (4H, m, phenyl), 7.42 (6H, m, phenyl), 7.13 (1H, s, 6-CH), 3.69 (8H, m, CH₂), 1.72 (3H, s, 5-Me), 1.43 (3H, s, CH₃-2), 1.32 (3H, s, CH₃-2), 1.12 (9H, s, ter-butyl);
Mass (FAB⁺) m/z: 522.2550 (M⁺+H), 465, 307, 154;
Anal. calcd for C₂₉H₃₈N₂O₅Si: C, 66.64; H, 7.33; N, 5.36. found: C, 66.50; H, 7.20; N, 5.29.

### (2) Production of 2,2-dimethyl-5-(thymine-9-yl-methyl)5-hydroxymethyl-1,3-dioxane

THF (10 ml) was added to and dissolved the 2,2-dimethyl-5-t-butyl-diphenyloxylmethyl-5-(thymine-9-yl-methyl)-1,3-dioxane (200 mg, 0.38 mmol) under Ar substitution. TBAF (3 ml) was added to the solution, and that mixture was agitated overnight.
The solvent was evaporated from that mixture, the residue that was obtained was purified by silica gel column chromatography (CHCl₃:MeOH = 100:1 to 20:1), yielding the title compound as a white crystal. (yield amount 101 mg, 0.35 mmol, 94% yield)

Mass (EI) m/z: 284.1372 (M⁺) 269, 233, 208, 195, 180;
Anal. calcd for C₁₃H₂ON₂O₅·1/5 H₂O: C, 54.23; H, 7.14; N, 9.73 found: C, 54.30; H, 6.90; N, 9.65.

### (3) Production of 1-(2,2-dihydroxymethyl-3-hydroxypropyl)thymine

30% TFA (10 ml) was added to the 2,2-dimethyl-5-(thymine-9-yl-methyl)5-hydroxymethyl-1,3-dioxane (48 mg, 0.166 mmol), and this mixture was agitated for approximately two hours. Water and methanol were added to the mixture, and then the solvent was removed from the mixture and hardened by drying under reduced pressure. The residue that was obtained was crystallized from a mixture of n-hexane and ether (n-hexane:Et₂O = 1:1), yielding the title compound as a white crystal. (yield amount 28 mg, 0.114 mmol, 67% yield)

Mass (EI) m/z: 244.1059 (M⁺) 195, 180, 152, 126, 96;
Anal. calcd for C₁₃H₂₀N₂O₅·1/5 H₂O: C, 48.46; H, 6.67; N, 11.30. found: C, 48.04; H, 6.27; N, 11.36.

### Example 6

Production of 1-(2,2-dihydroxymethyl-3-hydroxypropyl)5-fluorouracil

### (1) Production of 2,2-dimethyl-5-t-butyl-diphenylsilylmethyl-5-(5-fluorouracil-1-yl-methyl)-1,3-dioxane

A DMF (50 ml) solution of 2,2-dimethyl-5-t-butyl-diphenylsilyloxylmethyl-5-toluenesulfonylmethyl-1,3-dioxane (2.23 g, 3.92 mmol) was slowly added dropwise to a mixture of vacuum-dried 5-fluorouracil (0.765 g, 5.88 mmol), 60% NaH (0.235 g, 5.88 mmol) and 18-crown-6-ether (1.06 g, 3.99 mmol). The mixture that was obtained was agitated for 24 hours at approximately 85°C in an Ar atmosphere. A mixture of n-hexane and ethyl acetate (EtoAc:n-Hex = 1:1) and water was added to the mixture that was obtained, and extraction was performed. The organic layer that was obtained was washed with a saturated NaCl aqueous solution and a saturated NaHCO₃ aqueous solution in that order, and dried. The solvent was removed from the organic layer by evaporation under reduced pressure, and the residue that was obtained was purified by silica gel column chromatography (n-Hex:EtOAc = 20:1 to 0:1), yielding the title compound as a white crystal. (156 mg, 0.296 mmol, 8%)

¹HNMR (DMSO-d₆, 400 MHz) δ :11.8 (1H, s, NH), 7.81 (1H, d, J=6.4 Hz, CH-uracil-6), 7.66 (4H, m, Phe), 7.44 (6H, m, Phe), 3.67 (8H, m, CH₂-4,5,5,6), 1.31 (4H, m, CH₃-2), 1.18 (2H, m, CH₃-2), 1.00 (9H, s, t-butyl).

### (2) Production of 1-(2-t-butyl-diphenylsilylrnothyl-2-hydroxymethyl-3-hydroxypropyl)-5-fluorouracil

40 ml of 30% acetic acid was added to the 2,2-dimethyl-5-t-butyl-diphenylsllylmethyl-5-(5-fluorouracil-1-yl-methyl)-1,3-dioxane (151 mg, 0.287 mmol), and this mixture was heated for three hours at approximately 70°C. The solvent was evaporated from that mixture under reduced pressure, and the residue that was obtained was purified by silica gel column chromatography (CHCl₃:MeOH = 50:1 to 30:1), yielding the title compound as a colorless, transparent oil. (41.6 mg, 0.0855 mmol, 30%)

¹HNMR (CDCl₃, 400 MHz) δ: 9.29 (1H, s, NH), 7.64 (5H, m, Phe, CH-uracil-6), 7.44 (6H, m, Phe), 3.92 (2H, s, OH-2,3), 3.56 (2H, s, CH₂-2), 3.47 (2H, d, J=10.8 Hz, CH₂-1), 3.32 (4H, d, J=13.2 Hz, CH₂-2,3), 1.12 (9H, s, t-butyl).

### (3) Synthesis of 1-(2,2-dihydroxymethyl-3- hydroxypropyl)-5-fluorouracil

TBAF (0.15 ml) was added to a THF solution of the 1-(2-t-butyl-diphenylsilylmethyl-2-hydroxymethyl-3-hydroxypropyl)5-fluorouracil (41.6 mg, 0.0855 mmol), and this solution was agitated for two hours. The solvent was evaporated from this mixture under reduced pressure, and the residue that was obtained was purified by silica gel column chromatography (CHCl₃:MeOH = 50:1 to 10:1), yielding the title compound as a white crystal. (14.2 mg, 0.057 mmol, 67%)

¹HNMR (CDCl₃, 400 MHz) δ: 9.29 (1H, s, NH), 7.64 (5H, m, Phe, CH-uracil-6), 7.44 (6H, m, Phe), 3.92 (2H, s, OH-2,3), 3. 56 (2H, s, CH₂-2), 3.47 (2H, d, J=10.8 Hz, CH₂-1), 3.32 (4H, d, J=13.2 Hz, CH₂-2,3).

### Example 7

Production of 1-(2,2-dihydroxymethyl-3-hydroxypropyl)5-fluorocytosine

### (1) Production of 2,2-dimethyl-5-t-butyl-diphenylsilylmethyl-5-(5-fluorocytosine-1-yl-methyl)-1,3-dioxane

A DMF (35 ml) solution of 2,2-dimethyl-5-t-butyl-diphenylsilyloxylmethyl-5-toluenesulfonylmethyl-1,3-dioxane (1.67 g, 2.93 mmol) was slowly added dropwise to a mixture of vacuum-dried 5-fluorocytosine (0.568 g, 4.40 mmol), 60% NaH (0.176 g, 4.40 mmol) and 18-crown-6-ether (0.790 g, 2.99 mmol). The mixture that was obtained was agitated for 48 hours at approximately 75°C in an Ar atmosphere.
A mixture of n-hexane and ethyl acetate (ethyl acetate:hexane = 1:1) and water were added to the mixture that was obtained, and extraction was performed. The organic layer that was obtained was washed with a saturated NaCl aqueous solution and a saturated NaHCO₃ aqueous solution in that order, and dried. The solvent was removed from the organic layer by evaporation under reduced pressure, and then the residue that was obtained was purified by silica gel column chromatography (n-Hex:EtOAc = 10:1 to 0:1), yielding the title compound as a white crystal. (352 mg, 0.669 mmol, 23%)

¹HNMR (CDCl₃, 400 MHz) δ; 9.29 (1H, s, NH), 7.64 (5H, m, Phe, CH-uracil-6), 7.44 (6H, m, Phe), 3.92 (2H, s, OH-2,3), 3.56 (2H, s, CH₂-2), 3.47 (2H, d, J=10.8 Hz, CH₂-1), 3.32 (4H, d, J=13.2 Hz, CH₂-2,3), 1.12 (9H, s, t-butyl).

### (2) Production of 1-(2-t-butyl-diphenylsilylmethyl-2-hydroxymethyl-3-hydroxypropyl)5-fluorocytosine

30% acetic acid (50 ml) was added to the 2,2-dimethyl-5-t-butyldiphenylsilylmethyl-5-(5-fluorocytosine-1-yl-methyl)-1,3-dioxane (352 mg, 0.669 mmol), and this mixture was heated for four hours at approximately 70°C. The solvent was evaporated from that mixture under reduced pressure, and the residue that was obtained was purified by silica gel column chromatography (CHCl₃:MeOH = 50:1 to 30:1), yielding a compound as a colorless, transparent oil. n-hexane and diethyl ether were added to that compound to precipitate the crystal, yielding the title compound as a white crystal (171 mg, 0.351 mmol, 52%)

¹HNMR (CDCl₃, 400 MHz) δ; 9.29 (1H, s, NH), 7.64 (5H, m, Phe, CH-uracil-6), 7.44 (6H, m, Phe), 3.92 (2H, s, OH-2,3), 3. 56 (2H, s, CH₂-2), 3.47 (2H, d, J=10.8 Hz, CH₂-1), 3.32 (4H, d, J=13.2 Hz, CH₂-2,3), 1.12 (9H, s, t-butyl).

### (3) Production of 1-(2,2-dlhydroxymethyl-3-hydroxypropyl)-5-fluorocytosine

TBAF (0.70 ml) was added to a THF solution of the 1-(2-t-butyl-diphenylsilylmethyl-2-hydroxymethyl-3-hydroxypropyl)-5-fluorocytosine (171 mg, 0.351 mmol), and this mixture was agitated for two hours. The solvent was evaporated from this mixture under reduced pressure, and ethanol was added to the residue that was obtained to precipitate the crystal, yielding the title compound as a white crystal. (32.0 mg, 0.129 mmol, 37%)

¹HNMR (DMSO-d₆, 400 MHz) δ; 11.3 (1H, s, NH), 7.40 (1H, s, CH-uracil-6), 3.62 (3H, br, OH-2,2,3), 3.27 (8H, m, CH₂-4,5,5,6), 1.72 (3H, s, CH₃-thymine).

### Example 8

Production of 9-[2-(2-diaminoethoxy-N,N-diisopropylaminophosphinyloxymethyl-2-(4,4'-dimethoxytrityloxy)-3-tert-butyldiphenylsilyloxy)propyl]-N⁶-benzoyladenine

### (1) Production of 2,2-dimethyl-5-t-butyl-diphenylsilyloxymethyl-5-(N⁶-benzoyladenine-9-yl-methyl)-1,3-dioxane

The 2,2-dimethyl-5-t-butyl-diphenylsilyloxylmethyl-5-(adenine-9-yl-methyl)-1,3-dioxane (2130 mg, 4.01 mmol) that was obtained in Example 1 (4) was dissolved in pyridine (40 ml), and benzoyl chloride (560 µl, 4.81 mmol) was added dropwise to that solution while chilling with ice. After agitation for 16 hours at room temperature, methanol was added to that mixture to decompose excess reagent. Toluene was blended with the residue that was obtained, and this was concentrated under reduced pressure to remove the pyridine. Chloroform was added to the syrup that was obtained, and the chloroform layer was washed with successively 2 N hydrochloric acid, saturated sodium bicarbonate solution, and saturated saline solution. After drying with sodium sulfate, the chloroform layer was concentrated under reduced pressure. The residue that was obtained was purified by silica gel column chromatography (ethyl acetate:hexane = 2:1), yielding the title compound as a white powder (yield amount 1530 mg, 2.41 mmol, yield 60%).

¹H-NMR (400 MHz, CDCl₃) δ: 11.1 (s, 1H, NH), 8.66 (s, 1H, H-8), 8.30 (s, 1H, H-2), 8.05-7.41 (m, 15H, Ph-CO, 2Ph-Si), 4.37 (s, 2H, CH₂-N), 3.76 (2d, 4H, 2CH₂-O-C), 1.25, 1.21 (2s, 6H, 2CH₃-C), 0.97 (s, 9H, tert-butyl).

### (2) Production of 9-(2,2-hydroxymethyl-8-tert-butyldiphenylsilyloxypropyl) -N⁶-benzoyl- adenine

p-toluenesulfonate monohydrate (500 mg, 2.63 mmol) was added to a methanol (20 ml) solution of the 2,2-dimethyl-5-t-butyl-diphenylsilyloxymethyl-5-(N⁶-benzoyladenine-9-yl-methyl)-1,3-dioxane (1600 mg, 2.52 mmol), and this mixture was agitated for 48 hours at room temperature. After confirming the disappearance of the starting material in the mixture with TLC (chloroform:methanol = 10:1), triethylamine was added to this mixture to neutralize it. The residue that was obtained by condensing the mixture under reduced pressure was purified by silica gel column chromatography (chloroform:methanol = 60:1), yielding the title compound as a white powder (890 mg, 1.49 mmol, yield 59%).

¹H-NMR (400 MHz, CDCl₃) δ: 9.12 (brs, 1H, NH), 8.75 (s, 1H, H-8), 8.09 (s, 1H, H-2), 8.05-7.41 (m, 15H, Ph-CO, 2Ph-Si), 4.48 (s, 2H, CH₂-N), 3.61 (s, 2H, CH₂-O-Si), 3.45, 3.26 (2 brd, 4H, 2CH₂-OH), 1.14 (s, 9H, tert-butyl).

### (3) Production of 9-[2-Hydroxymethyl-2-(4,4'-dimethoxytrityloxyl)-3-tert-butyldiphenylsilyloxypropyl]-N⁶-benzoyl-adenine

Dimethoxytrityl chloride (1360 mg, 4.02 mmol) and dimethylaminopyridine (491 mg, 4.02 mmol) were added to a pyridine (20 ml) solution of the 9-(2,2-hydroxymethyl-3-tert-butyldiphenylsilyloxypropyl)-N⁶-benzoyladenine (800 mg, 1.34 mmol), and this mixture was agitated for 18 hours at room temperature. After confirming the disappearance of the starting material in the mixture with TLC, methanol was added to this mixture to decompose excess reagent. Toluene was blended with the residue that was obtained, and this was condensed under reduced pressure. The residue that was obtained was dissolved in ethyl acetate, and this ethyl acetate solution was washed with distilled water. After drying with sodium sulfate, the ethyl acetate layer was concentrated under reduced pressure. The residue that was obtained was purified by silica gel column chromatography (chloroform:methanol = 200:1→ 100:1), yielding the title compound as a white powder (680 mg, 0.76 mmol, yield 57%).

¹H-NMR (400 MHz, CDCl₃) δ: 8.61 (s, 1H, H-8), 8.04-6.76 (m, 30H, 2 Ph-OMe, Ph-C, Ph-CO, 2 Ph-Si, H-2, NH), 4.39 (s, 2H, CH₂N), 3.71-3.69 (m, 8H, CHz-O-Si, 2 MeO), 3.45-3.37 (m, 4H, CH2-O-DMTr, CH₂OH), 0.90 (s, 9H, tert-butyl).

### (4) Production of 9-[2-(2-diaminoethoxy-N,N-diisopropylaminophosphinyloxymethyl-2-(4,4'-dimethoxytrityloxy)-3-tert-butyldiphenylsilyloxy)propyl]-N⁶-benzoyladenine

Hunig's base (76 µl, 0.44 mmol) and phosphite reagent (33 µl, 0.148 mmol) were added to a dichloromethane (0.37 ml) solution of 9-[2-hydroxynaethyl-2-(4,4'-dimethoxytrityloxyl)-3-tert-butyldiphenylsilyl oxypropyl]-N⁶-benzoyl-adenine (66.1 mg, 73.6 *µ*mol). After confirming the completion of the reaction with TLC, a saturated NaHCO₃ aqueous solution was added to this mixture to stop the reaction. This mixture was separated with chloroform and saturated NaHCO₃ aqueous solution. The organic layer that was obtained was concentrated under reduced pressure, and that residue was purified by silica gel column chromatography (ethyl acetate:hexane = 2:1), yielding the title compound (44.4 mg, 44 *µ*mol, yield 60%).

³¹PNMR (400 MHz, DMSO-d₆) δ [ppm]: 147.47 ppm.

### Example 9

Production of a single strand oligonucleotide analog made from sequence number 1 (see the base sequence shown below) (it should be noted that the nucleoside analog has a tert-butyldiphenylsilyl group), in which a single nucleoside analog has been introduced into a center portion of the base sequence

Eq.30 5' - d(AAG GAAAA*G AGG AAA GA) - 3'

An oligonucleotide analog (DNA-type) was produced in accordance with the base sequence of sequence number 1 through a phosphoroamidite method that uses a nucleic acid autosynthesis device and a CPG resin. At the sequence shown by A* of the base sequence was introduced the 9-[2-(2-diaminoethoxy-N,N-diisopropylaminophosphinyloxymethyl-2-(4,4'dimethoxytrityloxy)-3-tert-butyldiphenylsilyloxy)propyl]-N⁶-benzoyl-adenine that was produced in Example 8 as a nucleoside monomer. For the rest of the sequence, deoxyribose-type nucleosides were used for introduction. 1 *µ*mol CPG resin for solid-phase synthesis was used, and each condensation time was one minute.

With the oligonucleotide analog of sequence number 1 that was linked to the CPG resin protected by a benzoyl group and a 1-tert-butyldiphenylsilyloxy group, synthesis by the nucleic acid autosynthesis device was finished.

The oligonucleotide linked to the CPG resin was reacted at 55°C for 12 hours in 28% ammonia aqueous solution (1.5 mL). The reaction mixture was concentrated under reduced pressure. TBAF solution (1 mL) was added to the concentrate that was obtained, and this mixture was agitated at room temperature for 12 hours to deprotect the silyl group. The mixture that was obtained subsequently was diluted with 0.1 M TEAA buffer solution (30 mL). This mixture was purified by C-18 reverse phase column chromatography (Sep-Pak) (eluent: 50% CH₃CN (2 mL) in water), yielding the target single-strand oligonucleotide analog. MALDI-TOF/MS calculated value: 5598.96, actual measurement value 5594.03

The 0.1 M TEAA buffer solution used in Example 9 was prepared as follows. First, water was added to a mixture of 2 N acetic acid (114.38 mL) and triethylamine (277.6 mL) to reach 1 L. Acetic acid was added to this solution to adjust the pH to 7.0, and then that solution was diluted by a dilution factor of 20 times to prepare the 0.1 M TEAA buffer solution.

### Reference Example 1

A single-strand oligonucleotide was obtained in the same manner as in Example 9, according to the base sequence of sequence number 2 (see the base sequence shown below) instead of sequence number 1.

Eq. 31 5' - d(TC TTT CCT CTT TTC CTT) - 3'

### Example 10

The oligonucleotide analog (0.8 nmmol) made from sequence number 1 that was produced in Example 9 was dissolved in an annealing buffer (10 mM sodium phosphate salt (pH 7.0) and 1 M NaCl). This solution was incubated for one minute at 90°C, then for one hour at 37°C, yielding a double-stranded oligonucleotide analog such as that with the base sequence shown below, composed of the oligonucleotide analog made from sequence number 1 and the oligonucleotide made from sequence number 2.

Eq.32 5' - d(AAG GAA AA* G AGG AAA GA) - 3' 3'- d(TTC CTT TTC TCC TTT CT) - 5'

### Comparative Example 1

A single-strand oligonucleotide analog was produced in the same manner as in Example 9, based on the base sequence of sequence number 3 (see the base sequence shown below) instead of sequence number 1.

Eq. 33 5' - d(AAG GAA AAG AGG AAA GA) - 3'

### Comparative Example 2

A double-stranded oligonucleotide analog such as that shown below, composed of the oligonucleotide made from sequence number 3 and the oligonucleotide made from sequence number 2, was obtained in the same manner as in Example 10, using the oligonucleotide made from sequence number 3, which was produced in Comparative Example 1, instead of the oligonucleotide analog made from sequence number 1, which was produced in Example 9.

Eq.34 5'- d(AAG GAA AAG AGG AAA GA)- 3' 3' - d(TTC CTT TTC TCC TTT CT) - 5'

### Stability of the Double Strand

The measured Tm values for the double-stranded oligonucleotide analog produced in Example 10 and the double-stranded oligonucleotide produced in Comparative Example 2 are shown in Table 1 below.

**Table 1**

| | Tm value (°C) |
|---|---|
| Example 10 | 49.1 |
| Comparative Example 2 | 57.7 |

It can be understood from Table 1 that an oligonucleotide analog in which one base has been substituted by the nucleoside analog of the invention (it should be noted that the nucleoside analog has a tert-butyldiphenylsilyl group) has the ability to form a double strand.

### Example 11

The oligonucleotide analog made from sequence number 1 that was produced in Example 9 (100 pmol) was mixed while chilling with ice into a mixture solution of 10xPNK buffer solution (2 *µ*L), 6 unit/*µ*L of T4 polynucleotide kinase (E. Coli A19) (1 *µ*L), *Y*⁻³²P ATP (1 *µ*L) and sterilized water (16 *µ*L), and then this was agitated for 30 minutes at 37°C. Impurities were then removed from the mixture using a spin column to yield an oligonucleotide analog made from sequence number 1 whose 5'-end is labeled with ³²P isotope.

### Comparative Example 3

An oligonucleotide made from sequence number 3, whose 5'-end is labeled with ³²P isotope, was obtained in the same manner as in Example 11, except that the oligonucleotide made from sequence number 3 that was produced in Comparative Example 1 was used instead, of the oligonucleotide analog made from sequence number 1 that was prepared in Example 9.

### Nuclease Resistance

Evaluation of the exonuclease resistance of the single-strand oligonucleotide analog
The exonuclease resistance of the single-strand oligonucleotide analog made from sequence number 1 that was obtained in Example 11, and of the natural single-strand oligonucleotide made from sequence number 3 that was obtained in Comparative Example 3, was evaluated. As the exonuclease, snake venom phosphorodiesterase (SVP) was used. SVP selectively cleaves phosphodiester bonds to degrade an oligonucleotide into 5'-monophosphate nucleotides. It should be noted that the 10 µM single-strand oligonucleotide analog solution is produced by adding the unmarked single-strand oligonucleotide analog (400 pmol) made from sequence number 1 that was produced in Example 9 to the single-strand oligonucleotide analog (100 pmol) made from sequence number 1 that was produced in Example 11, and this was adjusted to 10 µM using sterilized water. The 10 µM single-strand oligonucleotide solution also was prepared in the same manner as above.

From the reaction solution with the composition shown below, a sample of the reaction solution (5 µL) was taken in an Eppendorf tube that included a loading solution (7 M urea XC BPB; 5 µL) at 1, 5, 10, 15, and 30 minutes, and the reaction was stopped. It should be noted that the sample at 0 minutes has not had the SVP aqueous solution added to it. The samples attained at these times were electrophoresed by PAGE with 20% urea, and were separated in the gel. The gel was brought into contact with an imaging plate to transfer the separated image in the gel. This image was read using a bioimaging analyzer (trade name: BAS 2000 made by Fuji Photo Film) and image processed by RI image analysis software. The results are shown in FIG. 1.

Composition of the reaction solution of the single-strand oligonucleotide analog (Example 11)

| | |
|---|---|
| single strand oligonucleotide analog (final concentration 10 µM) | 4 µL |
| buffer solution (250 mM Tris-HCl, 50 mM MgCl₂ (pH 7.0)) | 6 µL |
| 1 units/mL SVP aqueous solution | 4 µL |
| sterilized water | 26 µL |
| total | 40 µL |

Composition of the reaction solution of a single-strand oligonucleotide (Comparative Example 3)

| | |
|---|---|
| single strand oligonucleotide (final concentration 10 µM) | 4 µL |
| buffer solution (250 mM Tris-HCl, 50 mM MgCl₂ (pH 7.0)) | 6 µL |
| 1 units/mL SVP aqueous solution | 4 µL |
| sterilized water | 26 µL |
| total | 40 µL |

From FIG. 1 it is confirmed that the single-strand oligonucleotide analog (here, the nucleoside analog has a tert-butyldlphenylsilyl group) has improved exonuclease resistance when compared to a natural single-strand oligonucleotide. It also was confirmed from FIG. 1 that the single-strand oligonucleotide analog demonstrates nuclease resistance at not only site 1 but also at site 2. Site 1 is the site of a bond between the nucleoside analog and a natural nucleoside. Site 2 is the site of a bond between natural nucleosides, however, one of the natural nucleoside bond sites is a bond with the nucleoside analog. Thus, it was confirmed that the nucleoside analog of the invention increases nuclease resistance not only at the site of bonds with adjacent nucleosides but also at the site of bonds between nucleosides at distant positions.

### Industrial Applicability

The nucleoside analog of the invention is useful as a nucleoside for producing an oligonucleotide for a test kit, for example.
Sequence List Free Text

| | |
|---|---|
| Sequence No. 1 | oligonucleotide analog |
| Sequence No. 2 | oligonucleotide |
| Sequence No. 3 | oligonucleotide |

## Claims

1. A nucleoside analog or salt thereof represented by Formula (I) below, 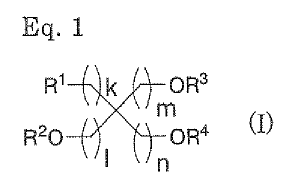 wherein, in Formula (I), R¹ is any group selected from the group consisting of the group of Formula (1) below, the group of Formula (1) below in which a functional group of Formula (1) has been protected by a protecting group, the group of Formula (2) below, the group of Formula (2) below in which a functional group of Formula (2) has been protected by a protecting group, the group of Formula (3) below, the group of Formula (3) below in which a functional group of Formula (3) has been protected by a protecting group, the group of Formula (4) below, the group of Formula (4) below in which a functional group of Formula (4) has been protected by a protecting group, the group of Formula (5) below, the group of Formula (5) below in which a functional group of Formula (5) has been protected by a protecting group, the group of Formula (6) below, the group of Formula (6) below in which a functional group of Formula (6) has been protected by a protecting group, the group of Formula (7) below, the group of Formula (7) below in which a functional group of Formula (7) has been protected by a protecting group, the group of Formula (8) below, and the group of Formula (8) below in which a functional group of Formula (8) has been protected by a protecting group;
R² is H or a protecting group;
R³ is H or a protecting group;
R⁴ is H or a solid-phase synthesis activating phosphate group; and
k, l, m, and n are each independently an integer from 1 to 10.

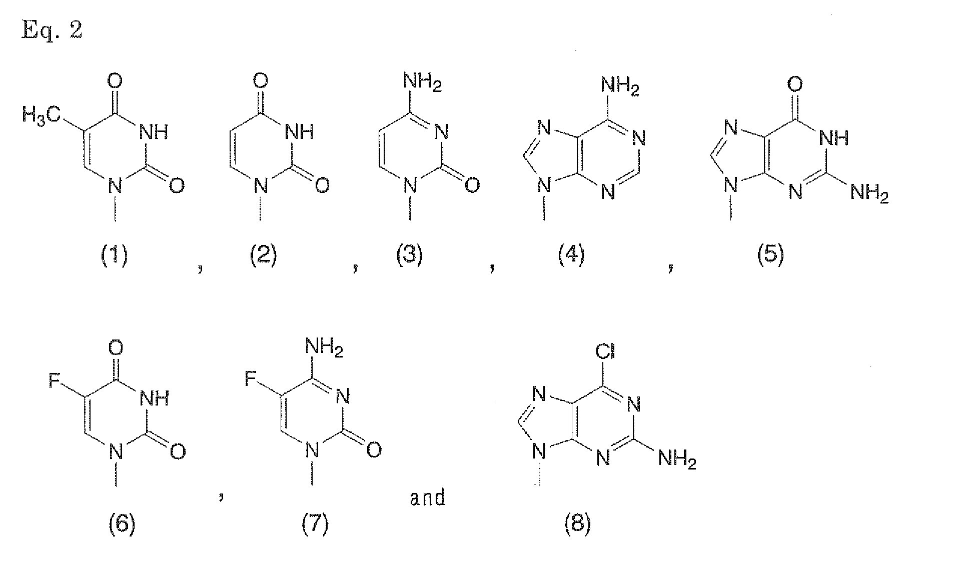

2. The nucleoside analog or salt thereof according to claim 1,
wherein
R² is H, 4,4'-dimethoxytrityl (DMTr), tert-butyldimethylsilyl (TBDMS), 4-monomethoxytrityl (MMTr), tert-butyldiphenylsilyl (TBDPS), or (9-phenyl)xanthene-9-yl;
R³ is H, 4,4'-dimethoxytrityl (DMTr), tert-butyldimethylsilyl (TBDMS), 4-monomethoxytrityl (MMTr), tert-butyldiphenylsilyl (TBDPS), or (9-phenyl)xanthene-9-yl; and
R⁴ is H or the group represented by Formula (10) below.

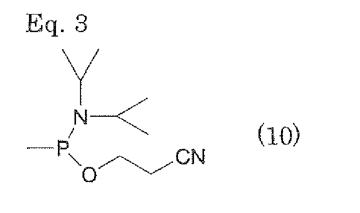

3. The nucleoside analog or salt thereof according to claim 1,
wherein, in Formula (I), R¹ is any group selected from the group consisting of the group of Formula (1), the group of Formula (2), the group of Formula (3), the group of Formula (4), the group of Formula (5), the group of Formula (6), the group of Formula (7), and the group of Formula (8); and
k, l, m, and n are each 1.

4. The nucleoside analog according to claim 1, selected from the group consisting of
a nucleoside analog represented by Formula (1), in which R¹ is the group of Formula (1) and R², R³, and R⁴ are each H,
a nucleoside analog represented by Formula (1), in which R¹ is the group of Formula (2) and R², R³, and R⁴ are each H,
a nucleoside analog represented by Formula (I), in which R¹ is the group of Formula (3) and R², R³, and R⁴ are each H,
a nucleoside analog represented by Formula (I), in which R¹ is the group of Formula (4) and R², R³, and R⁴ are each H,
a nucleoside analog represented by Formula (I), in which R¹ is the group of Formula (5) and R², R³, and R⁴ are each H,
a nucleoside analog represented by Formula (I), in which R¹ is the group of Formula (6) and R², R³, and R⁴ are each H,
a nucleoside analog represented by Formula (1), in which R¹ is the group of Formula (7) and R², R³, and R⁴ are each H, and
a nucleoside analog represented by Formula (I), in which R¹ is a group in which the functional group of the group of Formula (7) is protected by benzoyl, R² is 4,4'-dimethoxytrityl (DMTr), R³ is tert-butyldiphenylsilyl (TBDPS), and R⁴ is the group represented by Formula (10) below.

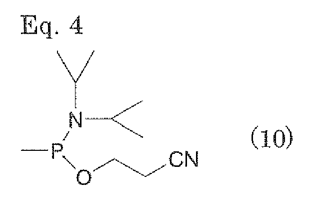

5. An oligonucleotide analog in which one or more of the nucleosides making up an oligonucleotide have been substituted by a nucleoside analog,
wherein the nucleoside analog is the nucleoside analog according to claim 1, in which, in Formula (I), R¹ is any group selected from the group consisting of the group of Formula (1), the group of Formula (2), the group of Formula (3), the group of Formula (4), the group of Formula (5), the group of Formula (6), the group of Formula (7), and the group of Formula (8), and R², R³, and R⁴ are each H.

6. The oligonucleotide analog according to claim 5,
wherein the oligonucleotide analog is a single-strand oligonucleotide.

7. The oligonucleotide analog according to claim 5,
wherein the oligonucleotide analog has an ability to form a double strand.

8. The oligonucleotide analog according to claim 5,
wherein the oligonucleotide analog is nuclease resistant.

9. A gene expression inhibiting agent including an oligonucleotide,
wherein the oligonucleotide is the oligonucleotide analog according to claim 5.

10. A pharmaceutical composition for treating diseases that result from expression of a gene, wherein the pharmaceutical composition includes the gene expression inhibiting agent according to claim 9.

11. A test kit that includes the oligonucleotide analog according to claim 5,
wherein the oligonucleotide analog is to be hybridized with a gene in a specimen to test the gene.

12. A method of inhibiting gene expression using an oligonucleotide,
wherein the oligonucleotide is the oligonucleotide analog according to claim 5.

13. A method of producing the nucleoside analog or salt thereof represented by Formula (II) below, 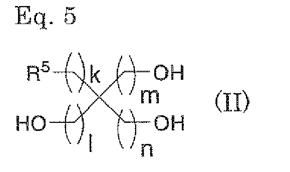 corresponding to Formula (I) according to claim 1 in which R¹ is any group selected from the group consisting of the group of Formula (1), the group of Formula (1) in which a functional group of Formula (1) has been protected by a protecting group, the group of Formula (2), the group of Formula (2) in which a functional group of Formula (2) has been protected by a protecting group, the group of Formula (3), the group of Formula (3) in which a functional group of Formula (3) has been protected by a protecting group, the group of Formula (4), the group of Formula (4) in which a functional group of Formula (4) has been protected by a protecting group, the group of Formula (5) in which a functional group of Formula (5) has been protected by a protecting group, the group of Formula (6), the group of Formula (6) in which a functional group of Formula (6) has been protected by a protecting group, the group of Formula (7), the group of Formula (7) in which a functional group of Formula (7) has been protected by a protecting group, the group of Formula (8), and the group of Formula (8) in which a functional group of Formula (8) has been protected by a protecting group; and
R², R³, and R⁴ are each H;
the method comprising:
reacting a compound represented by Formula (VII) below and a compound represented by Formula (VIII) below to yield a compound represented by Formula (IX) below; 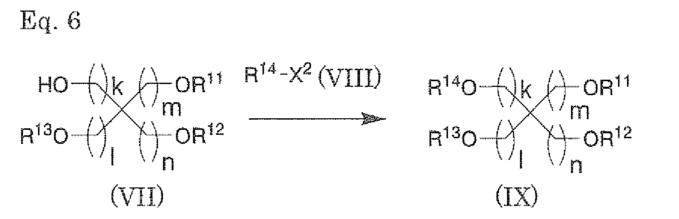
condensing the compound represented by Formula (IX) and a compound represented by Formula (X) below in the presence of a base to yield a compound represented by Formula (XI) below; and 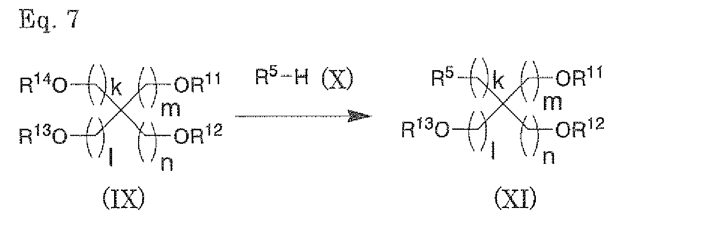
removing R¹¹, R¹², and R¹³ of the compound represented by Formula (XI), to yield the nucleoside analog or salt thereof represented by Formula (II);
wherein in the formulas, R⁵ is any group selected from the group consisting of the group of Formula (1) below, the group of Formula (1) below in which a functional group of Formula (1) has been protected by a protecting group, the group of Formula (2) below, the group of Formula (2) below in which a functional group of Formula (2) has been protected by a protecting group, the group of Formula (3) below, the group of Formula (3) below in which a functional group of Formula (3) has been protected by a protecting group, the group of Formula (4) below, the group of Formula (4) below in which a functional group of Formula (4) has been protected by a protecting group, the group of Formula (5) below in which a functional group of Formula (5) has been protected by a protecting group, the group of Formula (6) below, the group of Formula (6) below in which a functional group of Formula (6) has been protected by a protecting group, the group of Formula (7) below, the group of Formula (7) below, the group of Formula (8) below, and the group of Formula (8) below in which a functional group of Formula (8) has been protected by a protecting group; 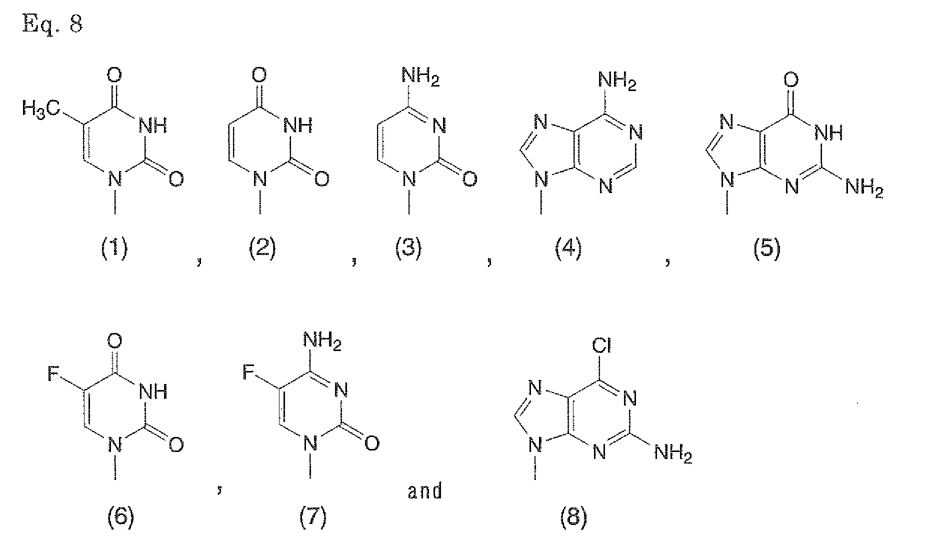
R¹¹ is a protecting group;
R¹² and R¹³ together are a group represented by the formula -CR¹⁵R¹⁶-;
R¹⁵ and R¹⁶ are each independently any one selected from the group consisting of a hydrogen atom, a lower alkyl group, and a lower alkoxyl group;
R¹⁴ is a group represented by the formula -SO₂- R¹⁷;
R¹⁷ is an aryl group that may be substituted with a lower alkyl;
X² is independently a halogen atom; and
k, l, m, and n are each independently an integer from 1 to 10.

14. A method of producing the nucleoside analog or salt thereof 1 represented by Formula (III) below,
corresponding to Formula (I) according to claim in which R¹ is the group of Formula (5), and R², R³, and R⁴ are each H; 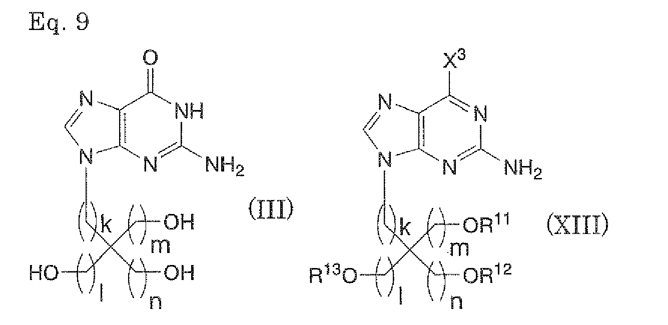 the method comprising
removing R¹¹, R¹², and R¹³ of the compound represented by Formula (XIII); and
performing hydrolysis to yield the nucleoside analog or salt thereof represented by Formula (III);
wherein in the formulas,
R¹¹ is a protecting group;
R¹² and R¹³ together are a group represented by the formula -CR¹⁵R¹⁶-;
R¹⁵ and R¹⁶ are each independently any one selected from the group consisting of a hydrogen atom, a lower alkyl group, and a lower alkoxyl group;
X³ is a halogen atom; and
k, l, m, and n are each independently an integer from 1 to 10.
